# EUROPEAN PATENT APPLICATION

(11) **EP 4 186 517 A1**
(43) Date of publication of application: **31.05.2023**
(21) Application number: 21744724.2
(22) Date of filing: 06.01.2021
(51) Int. Cl.: A61K 38/00, A61K 38/17, A61P 17/00, A61P 17/06, A61K 39/00

(54) **FUSION PROTEIN COMPRISING PD-L1 PROTEIN AND USE THEREOF**

(30) Priority: 23.01.2020 KR 20200008991
(71) Applicant: Genexine, Inc., Seongnam-si, Gyeonggi-do 13488 (KR)
(72) Inventor: SUNG, Young Chul, Seoul 04419 (KR); SHIN, Eun Ju, Yongin-si, Gyeonggi-do 16859 (KR); HWANG, Yuri, Seoul 07519 (KR); LEE, Sung Hee, Seoul 08797 (KR)
(74) Representative: dompatent von Kreisler Selting Werner - Partnerschaft von Patent- und Rechtsanwälten mbB
(86) International application number: PCT/KR2021/000123
(87) International publication number: WO 2021/149945

(57) **Abstract**

The present invention relates to a fusion protein including PD-L1 protein and a modified immunoglobulin Fc region and use thereof, and since the fusion protein has significantly higher purity and production yield compared to the existing fusion protein, has a high binding affinity to PD-1, reduces the proliferation of activated T cells, inhibits the generation of cytokines generated by activated T cells, and has an effect of inhibiting the infiltration of T cells or macrophages into tissues, it can be effectively used in the treatment of immune diseases.

## Description

### [Technical Field]

The present invention relates to a fusion protein including PD-L1 protein and a modified immunoglobulin Fc region and use thereof.

### [Background Art]

As a ligand for programmed death-1 (PD-1), human programmed cell death-ligand 1 (PD-L1) is a type 1 transmembrane protein that is expressed in hematopoietic cells such as T lymphocytes, B lymphocytes, dendritic cells or macrophages, as well as in non-hematopoietic cells such as keratinocytes, islet cells, hepatocytes and the like. Meanwhile, in order to activate T cells, in addition to the primary signal stimulation of the T cell receptor and antigen, the secondary signal stimulation (co-stimulation) is required at the same time. In this case, if there is no signal of either one, the T cell is in an inactive (anergy) state. As a secondary signaling factor (immune check point or immune modulator) that regulates the secondary signaling activity of T cells, programmed death-1 (PD-1) is capable of acting to inhibit T cell functions such as inhibiting the proliferation of T cells and reducing the expression of cytokines by binding to programmed cell death ligand 1 (PD-L1) or B7.1 (CD80) expressed on the cell surface, such as activated T cells (CD8 and/or CD4) or dendritic cells.

Binding between PD-1:PD-L1 is known to induce the activity of regulatory T cells (Immunol Rev. 2010 Jul; 236:219-42), and when PD-L1 protein (PD-L1-Ig) in which Fc of IgG1 was fused by using the immune tolerance-inducing function of PD-L1 was injected into a collagen-induced arthritis (CIA) mouse model, it was observed that the symptoms of arthritis were alleviated (Rheumatol Int. 2011 Apr; 31(4):513-9). Since PD-1 is expressed in activated T cells, PD-L1 protein is expected to be effectively used as a therapeutic agent that specifically targets active immune cells in autoimmune diseases as well as the induction of immune tolerance in organ transplantation.

Until now, therapeutic agents for the PD-1/PD-L1 cell signaling system have been developed in the direction of increasing T cell activity by inhibiting immune tolerance (tolerance breaking) as antagonists. However, an immunotherapeutic agent based on the induction of T cell immune tolerance using an agonist has not yet been developed. In the case of a PD-1/PD-L1 antagonist, it can be easily developed using antibody fusion technology, but it is not technically easy to develop a PD-1/PD-L1 signal agonist, which should be developed as a soluble protein.

The Fc fusion technology of immunoglobulin (Ig) is one of the techniques for increasing the *in vivo* half-life of protein therapeutics. However, since IgG1 used in the existing Ig fusion technology causes antibody dependent cell-mediated cytotoxicity (ADCC) and complement dependent cytotoxicity (CDC) in the body, Ig fusion proteins as therapeutic agents for autoimmune diseases or as immune tolerance inducers in organ transplantation do not play a role in suppressing the inflammatory response, but rather exacerbate inflammation.

Accordingly, the situation is that there is a need to develop a technique to increase the therapeutic efficacy of PD-L1 as an immunosuppressant by not inducing ADCC and CDC while maintaining the half-life of PD-L 1 similar to that of the existing Ig fusion protein therapeutics.

### [Disclosure]

### [Technical Problem]

An object of the present invention is to provide a fusion protein including programmed cell death-ligand 1 (PD-L1) protein and a modified immunoglobulin Fc region.

Another object of the present invention is to provide a nucleic acid molecule encoding the fusion protein.

Still another object of the present invention is to provide an expression vector including the nucleic acid molecule.

Still another object of the present invention is to provide a host cell including the expression vector.

Still another object of the present invention is to provide a pharmaceutical composition for preventing or treating immune disease, including a fusion protein including PD-L1 protein and a modified immunoglobulin Fc region as an active ingredient.

Still another object of the present invention is to provide the use of a fusion protein including PD-L1 protein and a modified immunoglobulin Fc region for producing a pharmaceutical preparation having an effect of preventing or treating immune disease.

Still another object of the present invention is to provide a method for preventing or treating immune disease, including administering a fusion protein including PD-L1 protein, a modified immunoglobulin Fc region and a pharmaceutically acceptable carrier to a subject.

### [Technical Solution]

In order to achieve the above objects, the present invention provides a fusion protein including PD-L1 protein and a modified immunoglobulin Fc region.

In addition, the present invention provides a nucleic acid molecule encoding the fusion protein.

In addition, the present invention provides an expression vector including the nucleic acid molecule.

In addition, the present invention provides a host cell including the expression vector.

In addition, the present invention provides a pharmaceutical composition for preventing or treating immune disease, including a fusion protein including PD-L1 protein and a modified immunoglobulin Fc region as an active ingredient.

In addition, the present invention provides the use of a fusion protein including PD-L1 protein and a modified immunoglobulin Fc region for producing a pharmaceutical preparation having an effect of preventing or treating immune disease.

In addition, the present invention provides a method for preventing or treating immune disease, including administering a fusion protein including PD-L1 protein, a modified immunoglobulin Fc region and a pharmaceutically acceptable carrier to a subj ect.

### [Advantageous Effects]

As a fusion protein in which PD-L1 protein and a modified immunoglobulin Fc region are linked by a sequence consisting of a GS sequence and an IgG1 hinge, the fusion protein according to the present invention is characterized in that it is prepared to maintain flexibility while inducing dimerization by being linked by a sequence consisting of a GS sequence and an IgG1 hinge. In addition, since the fusion protein according to the present invention has significantly higher purity and production yield compared to the existing fusion protein, has a high binding affinity to PD-1, reduces the proliferation of activated T cells, inhibits the generation of cytokines generated by activated T cells, and has an effect of inhibiting the infiltration of T cells or macrophages into tissues, it can be effectively used in the treatment of immune diseases.

### [Description of Drawings]

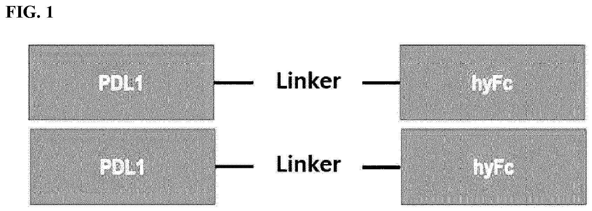

FIG. 1 shows the structure of a fusion protein (PD-L1-hyFc21 fusion protein) including PD-L1 protein and a modified immunoglobulin Fc region.
FIG. 2a is the results of analyzing the cell concentration of cells expressing the PD-L1-hyFc21 fusion protein over time, and FIG. 2b is the results of analyzing the purity of the target protein through SE-HPLC in the cell culture medium of cells expressing the PD-L1-hyFc21 fusion protein.
FIG. 3a is the results of analyzing the cell concentration of cells expressing the PD-L1-hyFc5 fusion protein over time, and FIG. 3b is the results of analyzing the purity of the target protein through SE-HPLC in the cell culture medium of cells expressing the PD-L1-hyFc5 fusion protein.
FIG. 4 is an SDS-PAGE analysis result of the purified PD-L1-hyFc21 or PD-L1-hyFc5 fusion protein.
FIG. 5a is an SE-HPLC analysis result of the purified PD-L1-hyFc21 fusion protein, and FIG. 5b is an SE-HPLC analysis result of the purified PD-L1-hyFc5 protein.
FIG. 6 is a gel IEF analysis result of the purified PD-L1-hyFc21 or PD-L1-hyFc5 fusion protein.
FIG. 7 is a differential scanning fluorescence (DSF) analysis result of the purified PD-L1-hyFc21 or PD-L1-hyFc5 fusion protein.
FIG. 8 is the results of comparing the binding affinity of the PD-L1-hyFc21 or PD-L1-hyFc5 fusion protein to PD-1.
FIG. 9 is the results of comparing the inhibition capacities of the mixed lymphocyte reaction by the PD-L1-hyFc21 or PD-L1-hyFc5 fusion protein.
FIG. 10a is the results of comparing the inhibition capacities of the proliferation of activated human CD4 T cells by the PD-L1-hyFc21 or PD-L1-hyFc5 fusion protein, and FIG. 10b is the results of comparing the inhibition capacities of the expression of cytokines of the activated human CD4 T cells.
FIG. 11 is the results of comparing the inhibition capacities of the expression of cytokines of activated mouse CD4 T cells by the PD-L1-hyFc21 or PD-L1-hyFc5 fusion protein.
FIG. 12a is the results of measuring the ear thicknesses of mice after subcutaneous administration of the PD-L1-hyFc21 fusion protein to the IMQ-induced psoriasis mouse model, and FIG. 12b is the results of measuring the ear thicknesses of mice after intravenous administration of the PD-L1-hyFc21 fusion protein to the IMQ-induced psoriasis mouse model.
FIG. 13a is the results of confirming the changes in the skin epithelial tissue through H&E staining after subcutaneous administration of the PD-L1-hyFc21 fusion protein to the rtTA-Peli1 psoriasis mouse model, and FIG. 13b is the results of measuring the thickness of the skin epithelial layer.
FIG. 14 is a result showing the degree of infiltration of T cells and macrophages by immunofluorescence analysis in a non-psoriasis control group (rtTA) or psoriasis-induced rtTA-Peli1 psoriasis mouse model (left panel: rtTA; and right panel: rtTA-Peli1).
FIG. 15 is the results of measuring the numbers of T cells and macrophages infiltrated into skin tissues after subcutaneous administration of the PD-L1-hyFc21 fusion protein to the rtTA-Peli1 psoriasis mouse model.
FIG. 16 is the results of measuring the number of K14⁺ keratinocytes in the skin tissue after subcutaneous administration of the PD-L1-hyFc21 fusion protein to the rtTA-Peli1 psoriasis mouse model.
FIG. 17 is the results of analyzing the changes in the skin epithelial layer as a score index after intravenous administration of the PD-L1-hyFc21 fusion protein to the rtTA-Peli1 psoriasis mouse model.
FIG. 18 is the results of measuring the skin thickness of the abdominal region after intravenous administration of the PD-L1-hyFc21 fusion protein to the rtTA-Peli1 psoriasis mouse model.
FIG. 19a is the results of confirming the changes in the skin epithelial tissue through H&E staining after intravenous administration of the PD-L1-hyFc21 fusion protein to the rtTA-Peli1 psoriasis mouse model, and FIG. 19b is the results of measuring the thickness of the skin epithelial layer.
FIG. 20 is the results of confirming the changes in the mRNA expressions of Th17 cell-associated genes (IL-17A and IL-22) and innate immune cell-associated genes (IL-1β and IL-24) through qRT-PCR after intravenous administration of the PD-L1-hyFc21 fusion protein to the rtTA-Peli1 psoriasis mouse model.

### [Best Mode]

Hereinafter, the present invention will be described in detail.

The present invention provides a fusion protein including PD-L1 protein and a modified immunoglobulin Fc region.

The PD-L1 protein may be an extracellular domain of PD-L1 protein or a fragment thereof. The extracellular domain of the PD-L1 protein may be a polypeptide including an immunoglobulin V like domain (Ig V like domain) of PD-L 1 and an immunoglobulin C like domain (Ig C like domain) of PD-L1.

Specifically, the extracellular domain of the PD-L1 protein is a protein region exposed outside the cell membrane, and may be a polypeptide consisting of the 19^{th} to 238^{th} amino acids of SEQ ID NO: 1 or a polypeptide consisting of the 19^{th} to 239^{th} amino acids of SEQ ID NO: 1.

In this case, the extracellular domain of the PD-L1 protein includes an Ig V like (Ig V, Ig V like) sequence that is a conserved sequence similar to the amino acid sequence of an immunoglobulin (Ig, immunoglobulin), and the highly conserved Ig V like sequence is the amino acid sequence of the 68^{th} to 114^{th} amino acids of SEQ ID NO: 1. In addition, it includes an Ig C like (Ig C, Ig C like) sequence, and the highly conserved sequence region is the amino acid sequence of the 153^{rd} to 210^{th} amino acids of SEQ ID NO: 1. In addition, the fragment of the extracellular domain of the PD-L1 protein may include all or a part of the Ig V like domain including the Ig V like sequence of PD-L1.

In addition, the Ig V like domain in the extracellular domain of the PD-L1 protein is a site capable of interacting with PD-1, and may be a polypeptide (SEQ ID NO: 3) consisting of the amino acid sequences of the 19^{th} to 239^{th} amino acids of SEQ ID NO: 1 or a polypeptide consisting of the amino acid sequence of the 21^{st} to 239^{th} amino acids of SEQ ID NO: 1. In addition, it may be a polypeptide (SEQ ID NO: 4) consisting of the amino acid sequence of the 19^{th} to 133^{rd} amino acids of SEQ ID NO: 1 or a polypeptide consisting of the amino acid sequence of the 21^{st} to 133^{rd} amino acids of SEQ ID NO: 1. In addition, it may be a polypeptide consisting of the amino acid sequence of the 21^{st} to 114^{th} amino acids of SEQ ID NO: 1 or a polypeptide consisting of the amino acid sequence of the 19^{th} to 114^{th} amino acids of SEQ ID NO: 1. In addition, it may be a polypeptide consisting of the amino acid sequence of the 21^{st} to 120^{th} amino acids of SEQ ID NO: 1 or a polypeptide consisting of the amino acid sequence of the 19^{th} to 120^{th} amino acids of SEQ ID NO: 1. In addition, it may be a polypeptide (SEQ ID NO: 5) consisting of the amino acid sequence of the 19^{th} to 127^{th} amino acids of SEQ ID NO: 1 or a polypeptide (SEQ ID NO: 6) consisting of the amino acid sequence of the 21^{st} to 127^{th} amino acids of SEQ ID NO: 1. In addition, it may be a polypeptide consisting of the amino acid sequence of the 21^{st} to 130^{th} amino acids of SEQ ID NO: 1 or a polypeptide consisting of the amino acid sequence of the 19^{th} to 130^{th} amino acids of SEQ ID NO: 1. In addition, it may be a polypeptide consisting of the amino acid sequence of the 21^{st} to 131^{st} amino acids of SEQ ID NO: 1 or a polypeptide consisting of the amino acid sequence of the 19^{th} to 131^{st} amino acids of SEQ ID NO: 1.

In addition, when the fragment of the extracellular domain of the PD-L1 protein includes an Ig V like domain or a fragment thereof, it may further include an immunoglobulin C like domain (Ig C like domain) of the extracellular domain of the PD-L1 protein. The Ig C like domain may be a polypeptide consisting of the amino acid sequence of the 133^{rd} to 225^{th} amino acids of SEQ ID NO: 1 or a polypeptide consisting of the amino acid sequence of the 134^{th} to 225^{th} amino acids of SEQ ID NO: 1.

In addition, when the fragment of the extracellular domain of the PD-L1 protein includes the Ig V like domain or a fragment thereof, it may further include a polypeptide or a fragment thereof including the Ig C like domain of the extracellular domain of the PD-L 1 protein. The polypeptide including the Ig C like domain refers to the extracellular domain of the PD-L1 protein excluding the Ig V domain, and it may be a polypeptide having the 134^{th} to 239^{th} amino acids of SEQ ID NO: 1 (SEQ ID NO: 7) or a polypeptide having the 134^{th} to 238^{th} amino acids of SEQ ID NO: 1 (SEQ ID NO: 8).

In addition, the extracellular domain of the PD-L1 protein or a fragment thereof may be derived from a human or a mouse.

The extracellular domain of the human PD-L1 protein is a polypeptide (SEQ ID NO: 3) consisting of the amino acid sequence of the 19^{th} to 239^{th} amino acids of SEQ ID NO: 1, and the extracellular domain of the mouse PD-L1 protein is a polypeptide consisting of the amino acid sequence of the 19^{th} to 239^{th} amino acids of SEQ ID NO: 2. In addition, the extracellular domain of the PD-L1 protein may have about 70%, 75%, 80%, 85%, 90%, 91%, 92%, 93%, 94%, 95%, 96%, 97%, 98%, 99% or more homology to a polypeptide sequence consisting of the amino acid sequence of the 19^{th} to 239^{th} amino acids of SEQ ID NO: 1.

Specifically, the human PD-L1 protein has 290 amino acid residues and includes the amino acid sequence of SEQ ID NO: 1 (Accession Number: Q9NZQ7). In the amino acid sequence of SEQ ID NO: 1, the 1^{st} to 18^{th} amino acid residues at the N-terminus are signal sequences, and the mature human PD-L1 protein includes the amino acid sequence of the 19^{th} to 290^{th} amino acids of SEQ ID NO: 1. The extracellular domain of the human PD-L1 protein includes the amino acid sequence of the 19^{th} to 238^{th} amino acids of SEQ ID NO: 1 or the 19^{th} to 239^{th} amino acids of SEQ ID NO: 1.

The human PD-L1 protein includes an Ig V like domain which is the 19^{th} to 127^{th} amino acids of SEQ ID NO: 1 and an Ig C like domain which is the 134^{th} to 226^{th} amino acids of SEQ ID NO: 1.

The mouse PD-L1 protein is reported to contain 290 amino acids, and it includes the amino acid sequence of SEQ ID NO: 2 (Accession Number: Q9EP73). The 1^{st} to 18^{th} amino acid residues of SEQ ID NO: 2 are signal sequences, and the mature mouse PD-L1 protein includes the amino acid sequence of the 19^{th} to 290^{th} amino acids of SEQ ID NO: 2. The extracellular domain of the mouse PD-L1 protein includes the amino acid sequence of the 19^{th} to 239^{th} amino acids of SEQ ID NO:2. The mouse PD-L1 protein includes an Ig V like protein having the 19^{th} to 127^{th} amino acids of SEQ ID NO: 2 and an Ig C like domain having the 133^{rd} to 224^{th} amino acids of SEQ ID NO: 2.

The extracellular domain of the PD-L1 protein may include the entirety of an Ig V like domain or a fragment thereof. In addition, the fragment of the extracellular domain of the PD-L1 protein may further include an Ig C like domain or a polypeptide including an Ig C like domain (the extracellular domain of PD-L1 excluding the Ig V like domain).

The extracellular domain of the PD-L1 protein or a fragment thereof may include variously modified proteins or peptides. The modification may be performed by substituting, deleting or adding one or more proteins to the wild-type PD-L1 protein as long as the function of PD-L1 is not altered. These various proteins or peptides may have 70%, 75%, 80%, 85%, 90%, 91%, 92%, 93%, 94%, 95%, 96%, 97%, 98% or 99% homology to the wild-type protein.

As used herein, the term "extracellular domain of PD-L1 protein" is also used as a concept including "the extracellular domain of PD-L1 protein and a fragment thereof."

As used herein, the terms "protein", "polypeptide" and "peptide" may be used interchangeably unless otherwise specified.

As used herein, the terms "PD-L1 fusion protein" and "PD-L1-modified immunoglobulin Fc region fusion protein" refer to a fusion protein in which PD-L1 protein, the extracellular domain of PD-L1 protein or a fragment thereof is linked to a modified immunoglobulin Fc region.

In the present invention, the PD-L1 protein may be fused to the N-terminus or C-terminus of a modified immunoglobulin Fc region, and preferably, the PD-L1 protein may be fused to the N-terminus of a modified immunoglobulin Fc region. The PD-L1 protein may be linked to the immunoglobulin Fc region by a linker peptide. The linker may include GGGSGGS (SEQ ID NO: 10), AAGSGGGGGSGGGGSGGGGS (SEQ ID NO: 17), GGSGG (SEQ ID NO: 18), GGSGGSGGS (SEQ ID NO: 19), GGGSGG (SEQ ID NO: 20), (G4S)n (n is an integer from 1 to 10), (GGS)n (n is an integer from 1 to 10), (GS)n (n is an integer from 1 to 10), (GSSGGS)n (n is an integer from 1 to 10), KESGSVSSEQLAQFRSLD (SEQ ID NO: 21), EGKSSGSGSESKST (SEQ ID NO: 22), GSAGSAAGSGEF (SEQ ID NO: 23), (EAAAK)n (n is an integer from 1 to 10), CRRRRRREAEAC (SEQ ID NO: 24), A(EAAAK)4ALEA(EAAAK)4A, GGGGGGGG (SEQ ID NO: 25), GGGGGG (SEQ ID NO: 26), AEAAAKEAAAAKA (SEQ ID NO: 27), PAPAP (SEQ ID NO: 28), (Ala-Pro)n (n is an integer from 1 to 10), VSQTSKLTRAETVFPDV (SEQ ID NO: 29), PLGLWA (SEQ ID NO: 30), TRHRQPRGWE (SEQ ID NO: 31), AGNRVRRSVG (SEQ ID NO: 32), RRRRRRRR (SEQ ID NO: 33), GFLG (SEQ ID NO: 34), GSSGGSGSSGGSGGGDEADGSRGSQKAGVDE (SEQ ID NO: 35) and the like. Preferably, PD-L1 and the immunoglobulin Fc region may be linked by a linker peptide consisting of the amino acid sequence of GGGSGGS (SEQ ID NO: 10). When the PD-L1 protein and the immunoglobulin Fc region are linked using the linker peptide, the activity, stability and productivity of the fusion protein may be optimized.

In addition, the fusion protein may exist in a dimer form. The bond between the fusion proteins constituting the dimer may be formed by a disulfide bond by a cysteine present in a linker. The fusion proteins constituting the dimer are identical. That is, the dimer may be a homodimer. In this case, the fusion protein may be soluble, and particularly, it may be dissolved in purified water or physiological saline.

The Fc region of the modified immunoglobulin may be any one of Fc regions of IgG1, IgG2, IgG3, IgD and IgG4, or a combination thereof. The Fc region is modified such that binding to the Fc receptor and/or complement does not occur. In particular, the Fc region of the modified immunoglobulin includes a hinge region, a CH2 domain and a CH3 domain from an N-terminal to C-terminal direction, wherein the hinge region may include a human IgG1 hinge region (SEQ ID NO: 16), wherein the CH2 domain may include a portion of the amino acid residues of the CH2 domain of human IgD and human IgG4, and wherein the CH3 domain may include a portion of the amino acid residues of the CH3 domain of human IgG4.

As used herein, the terms "Fc region", "Fc fragment" or "Fc" include the heavy chain constant region 2 (CH2) and heavy chain constant region 3 (CH3) of an immunoglobulin, and refer to proteins that include the variable regions of the heavy and light chains of an immunoglobulin and do not include the light chain constant region 1 (CL1). It may further include a hinge region of the heavy chain constant region. Hybrid Fc or hybrid Fc fragments are also referred to herein as "hFc" or "hyFc."

In addition, the Fc fragment of the present invention may be in the form of a native sugar chain, an increased sugar chain compared to the native form, a reduced sugar chain compared to the native form, or a form in which the sugar chain is removed. Immunoglobulin Fc sugar chains may be modified by conventional methods such as chemical methods, enzymatic methods and genetic engineering methods using microorganisms. Removal of sugar chains from the Fc fragment sharply reduces the binding affinity of the primary complement components C1 to C1q and results in a decrease or loss of ADCC or CDC, thereby not inducing an unnecessary immune response *in vivo.* In this regard, the immunoglobulin Fc fragment in a deglycosylated or aglycosylated form may be more suitable for the purpose of the present invention as a drug carrier. As used herein, the term "deglycosylation" refers to the enzymatic removal of sugars from an Fc fragment. In addition, the term "aglycosylation" means that the Fc fragment is produced in an unglycosylated form by prokaryotes, preferably *E. coli.*

In an exemplary embodiment of the present invention, the modified immunoglobulin Fc region may consist of the amino acid sequence of SEQ ID NO: 11 (hereinafter, "hyFc").

In an exemplary embodiment of the present invention, the fusion protein may be represented by Structural Formula I below.

N' - X - L - Y - C' (Structural Formula I)

In the above,
N' is the N terminus of the fusion protein, and C' is the C terminus of the fusion protein;
the X is PD-L1 protein, an extracellular domain of PD-L1 protein or a fragment thereof;
L is a linker; and
Y is an immunoglobulin Fc region.

Preferably, the fusion protein may consist of the amino acid sequence of SEQ ID NO: 12 or SEQ ID NO: 13. In addition, the fusion protein of the present invention may have about 70%, 75%, 80%, 85%, 90%, 91%, 92%, 93%, 94%, 95%, 96%, 97%, 98%, 99% or more homology to the amino acid sequence of SEQ ID NO: 12.

In addition, the present invention provides a nucleic acid molecule encoding the fusion protein.

Preferably, the nucleic acid molecule may be a nucleic acid molecule encoding a polypeptide consisting of the amino acid sequence of SEQ ID NO: 12 or SEQ ID NO: 13. In addition, the nucleic acid molecule may additionally include a signal sequence (or signal peptide) or a leader sequence.

As used herein, the term "signal sequence (or signal peptide)" refers to a short peptide present at the N-terminus of a newly synthesized protein classified as the secretory pathway. Signal sequences useful in the present invention include an antibody light chain signal sequence, such as antibody 1418 (Gillies et al., J Immunol Meth 1989 125: 191-202), an antibody heavy chain signal sequence, such as the MOPC141 antibody heavy chain signal sequence (Sakano et al., Nature 1980 286: 676-683), and other signal sequences known in the art (refer to, for example, Watson et al., Nucleic Acid Research 1984 12:5145-5164).

The signal peptide is well known in the art for its characterization, and it is generally known to include 16 to 30 amino acid residues and may contain more or fewer amino acid residues. A typical signal peptide consists of three regions of a basic N-terminal region, a central hydrophobic region and a more polar C-terminal region.

The central hydrophobic region includes 4 to 12 hydrophobic residues that anchor the signal sequence through the membrane lipid bilayer during migration of the immature polypeptide. After initiation, the signal sequence is cleaved in the lumen of the ER by cellular enzymes commonly known as signal peptidases. In this case, the signal sequence may be a secretion signal sequence of tissue plasminogen activation (tPa), HSV gDs or growth hormone. Preferably, the secretion signal sequence used in higher eukaryotic cells, including mammals and the like, may be used, and more preferably, the tPa sequence or the amino acid sequence of the 1^{st} to 18^{th} amino acids of SEQ ID NO: 1 may be used. In addition, the signal sequence of the present invention may be used by substituting with a codon having a high expression frequency in the host cell.

In addition, the present invention provides an expression vector including the nucleic acid molecule.

As used herein, the term "vector" is understood as a nucleic acid means including a nucleotide sequence that can be introduced into a host cell to be recombined and inserted into the host cell genome, or can replicate spontaneously as an episome. The vector includes linear nucleic acids, plasmids, phagemids, cosmids, RNA vectors, viral vectors and analogs thereof. Examples of viral vectors include, but are not limited to, retroviruses, adenoviruses, and adeno-associated viruses.

In the present invention, a useful expression vector may be RcCMV (Invitrogen, Carlsbad) or a variant thereof. Useful expression vectors include the human cytomegalovirus (CMV) promoter to promote continuous transcription of the gene of interest in mammalian cells, and the bovine growth hormone polyadenylation signal sequence to increase the steady-state level of RNA after transcription. In an exemplary embodiment of the present invention, the expression vector is pAD15, which is a modified vector of RcCMV

As used herein, the term "host cell" refers to prokaryotic and eukaryotic cells into which recombinant expression vectors can be introduced.

In the present invention, an appropriate host cell may be transformed or transfected with the DNA sequence of the present invention, and may be used for the expression and/or secretion of a target protein. Presently preferred host cells that can be used in the present invention include immortal hybridoma cells, NS/0 myeloma cells, 293 cells, Chinese hamster ovary cells (CHO cells), HeLa cells, CapT cells (human amniotic fluid derived cells) and COS cells.

As used herein, the terms "transformation" and "transfection" refer to the introduction of a nucleic acid (e.g., a vector) into a cell by a number of techniques known in the art.

In addition, the present invention provides a composition for preventing or treating immune disease, including a fusion protein including PD-L1 protein and a modified immunoglobulin Fc region as an active ingredient.

The immune disease may be a disease selected from the group consisting of autoimmune diseases, inflammatory diseases and transplantation rejection diseases of cells, tissues or organs.

The autoimmune disease may be selected from the group consisting of arthritis [acute arthritis, chronic rheumatoid arthritis, gouty arthritis, acute gouty arthritis, chronic inflammatory arthritis, degenerative arthritis, infectious arthritis, Lyme arthritis, proliferative arthritis, psoriatic arthritis, vertebral arthritis and rheumatoid arthritis such as juvenile-onset rheumatoid arthritis, osteoarthritis, arthritis chronica progrediente, arthritis deformans, polyarthritis chronica primaria, reactive arthritis and ankylosing spondylitis], psoriasis such as inflammatory hyperproliferative skin diseases, plaque psoriasis, gutatte psoriasis, pustular psoriasis and psoriasis of the nails, dermatitis including contact dermatitis, chronic contact dermatitis, allergic dermatitis, allergic contact dermatitis, herpetiformis dermatitis and atopic dermatitis, X-linked hyper-IgM syndrome, urticaria such as chronic allergic urticaria and chronic idiopathic urticaria, including chronic autoimmune urticaria, polymyositis/dermatomyositis, juvenile dermatomyositis, toxic epidermal necrolysis, scleroderma (including systemic scleroderma), systemic sclerosis, sclerosis including multiple sclerosis (MS) such as spino-optical MS, primary progressive MS (PPMS) and relapsing remitting MS (RRMS), progressive systemic sclerosis, atherosclerosis, arteriosclerosis, sclerosis disseminata and ataxic sclerosis, inflammatory bowel disease (IBD) [*e.g.,* Crohn's disease, autoimmune-mediated gastrointestinal disease, colitis such as ulcerative colitis, colitis ulcerosa, microscopic colitis, collagenous colitis, colitis polyposa, necrotizing enterocolitis, transmural colitis and autoimmune inflammatory bowel disease], pyoderma gangrenosum, erythema nodosum, primary sclerosing cholangitis (episcleritis), respiratory distress syndrome including adult or acute respiratory distress syndrome (ARDS), meningitis, inflammation of all or part of the uvea, iritis, choroiditis, autoimmune hematological disorder, rheumatoid spondylitis, acute hearing loss, IgE-mediated disease such as anaphylaxis and allergic and atopic rhinitis, encephalitis such as Rasmussen's encephalitis and limbic and/or brainstem encephalitis, uveitis such as anterior uveitis, acute anterior uveitis, granulomatous uveitis, nongranulomatous uveitis, phacoantigenic uveitis, posterior uveitis or autoimmune uveitis, glomerulonephritis (GN) with or without nephrotic syndrome such as chronic or acute glomerulonephritis such as primary GN, immune-mediated GN, membranous GN (membranous nephropathy), idiopathic membranous nephropathy or idiopathic membranous GN, membrano- or membranous proliferative GN (MPGN) including Type I and Type II and rapidly progressive GN, allergic diseases, allergic reaction, eczema including allergic or atopic eczema, asthma such as asthma bronchiale, bronchial asthma and autoimmune asthma, disease related with T cell infiltration and chronic inflammatory response, chronic pulmonary inflammatory disease, autoimmune myocarditis, leukocyte adhesion deficiency, systemic lupus erythematosus (SLE) or systemic lupus erythematosus such as cutaneous SLE, subacute cutaneous lupus erythematosus, neonatal lupus syndrome (NLE), lupus erythematosus disseminatus, lupus [including nephritis, cerebritis, pediatric, non-renal, extra-renal, discoid and alopecia], juvenile-onset (Type I) diabetes mellitus including pediatric insulin-dependent diabetes mellitus (IDDM), adult-onset (Type II) diabetes mellitus, autoimmune diabetes mellitus, idiopathic diabetes insipidus, immune responses associated with acute and delayed hypersensitivity mediated by T-lymphocytes and cytokines, granulomatosis including tuberculosis, sarcoidosis, lymphomatoid granulomatosis, Wegener's granulomatosis, agranulocytosis, vasculitis [including giant vessel vasculitis (polymyalgia rheumatic and Takayasu's arteritis], Kawasaki disease, medium vessel vasculitis including polyarteritis nodosa, microscopic polyarteritis, CNS arthritis, necrotizing, cutaneous or hypersensitivity vasculitis, systemic necrotizing vasculitis, vasculitides including ANCA-related vasculitis such as Churg-Strauss vasculitis or syndrome (CSS), temporal arteritis, aplastic anemia, autoimmune aplastic anemia, coombs benign anemia, Diamond Blackfan anemia, immune-hemolytic anemia including hemolytic anemia or autoimmune hemolytic anemia (AIHA), pernicious anemia (anemia perniciosa), Addison's disease, pure red cell anemia aplasia (PRCA), factor VHI deficiency, hemophilia A, autoimmune neutropenia, pancytopenia, leukopenia, leukocyte diapedesis-related disease, CNS inflammatory disorder, multiple organ injury syndrome such as those secondary to septicemia, trauma or hemorrhage, antigen-antibody complex-mediated diseases, anti-glomerular basement membrane disease, anti-phospholipid antibody syndrome, allergic neuritis, Bechet's or Behcet's disease, Castleman's syndrome, Goodpasture's syndrom, Raynaud's syndrome, Sjogren's syndrome, Stevens-Johnson syndrome, pemphigoid such as bullous pemphigoid and skin pemphigoid, pemphigus (including pemphigus vulgaris, pemphigus foliaceus, pemphigus mucous-membrane pemphigoid and pemphigus erythematosus), autoimmune polyendocrinopathies, Reiter's disease or syndrome, immune complex nephritis, antibody-mediated nephritis, neuromyelitis optica, polyneuropathies, chronic neuropathy such as IgM polyneuropathy or IgM-mediated neuropathy, thrombocytopenia (e.g., one which develops in myocardial infarction patient), including thrombotic thrombocytopenic purpura (TTP) and autoimmune or immune-mediated thrombocytopenia such as idiopathic thrombocytopenic purpura (ITP) including chronic or acute ITP, autoimmune disorder of the testis and ovary including autoimmune orchitis and oophoritis, primary hypothyroidism, hypothyroidism including thyroiditis such as autoimmune thyroiditis, autoimmune endocrine disease, Hashimoto's disease, chronic thyroiditis (Hashimoto's thyroiditis) or subacute thyroiditis, autoimmune thyroid disease, idiopathic hypothyroidism, Grave's disease, polyglandular syndrome such as autoimmune polyglandular syndrome (or polyglandular endocrinopathy syndrome), paraneoplastic syndromes including paraneoplastic neurological syndrome such as Lambert-Eaton myasthenic syndrome or Eaton-Lambert syndrome, stiff-man or stiff-person syndrome, encephalomyelitis such as allergic encephalomyelitis or encephalomyelitis allergica and experimental allergic encephalomyelitis (EAE), myasthenia gravis such as thymoma-associated myasthenia gravis, cerebellar degeneration, neuromyotonia, opsoclonus or opsoclonus myoclonus syndrome (OMS) and sensory neuropathy, multifocal motor neuropathy, Sheehan's syndrome, autoimmune hepatitis, chronic hepatitis, lupoid hepatitis, giant cell hepatitis, chronic active hepatitis or autoimmune chronic active hepatitis, lymphoid interstitial pneumonitis, bronchiolitis obliterans (non-transplant) vs. NSIP, Guillain-Barre syndrome, Berger's disease (IgA nephropathy), idiopathic IgA nephropathy, linear IgA dermatosis, primary biliary cirrhosis, pneumonocirrhosis, autoimmune enteropathy syndrome, Celiac disease, Coeliac disease, celiac sprue (gluten enteropathy), refractory sprue, idiopathic sprue, cryoglobulinemia, amyotrophic lateral sclerosis (ALS, Lou Gehrig's disease), coronary artery disease, autoimmune ear disease such as autoimmune inner ear disease (AGED), autoimmune hearing loss, opsoclonus myoclonus syndrome (OMS), polychondritis such as refractory or relapsing polychondritis, pulmonary alveolar proteinosis, amyloidosis, scleritis, non-cancerous lymphocytosis, primary lymphocytosis including monoclonal B cell lymphocytosis (e.g., benign monoclonal gammopathy and monoclonal gammopathy of undetermined significance; MGUS), peripheral neuropathy, paraneoplastic syndrome, epilepsy, migraine, arrhythmia, muscular disorder, deafness, blindness, periodic paralysis, channelopathies such as CNS channelopathies, autism, inflammatory myopathy, focal segmental glomerulosclerosis (FSGS), endocrine ophthalmopathy, uveoretinitis, chorioretinitis, autoimmune hepatological disorder, fibromyalgia, multiple endocrine failure, Schmidt's syndrome, adrenalitis, gastric atrophy, presenile dementia, demyelinating diseases such as autoimmune demyelinating diseases, diabetic nephropathy, Dressler's syndrome, alopecia areata, CREST syndrome (calcinosis), Raynaud's phenomenon, esophageal dysmotility, sclerodactyly and telangiectasia, male and female infertility, mixed connective tissue disease, Chagas' disease, rheumatic fever, recurrent abortion, farmer's lung, erythema multiforme, post-cardiotomy syndrome, Cushing's syndrome, bird-fancier's lung, allergic granulomatous angiitis, benign lymphocytic angiitis, Alport's syndrome, alveolitis such as allergic alveolitis and fibrous periostitis, interstitial lung disease, transfusion diseases, leprosy, malaria, leishmaniasis, trypanosomiasis, schistosomiasis, ascariasis, aspergillosis, Samter's syndrome, Caplan's syndrome, dengue, endocarditis, endomyocardial fibrosis, diffuse interstitial pulmonary fibrosis, interstitial lung fibrosis, idiopathic pulmonary fibrosis, cystic fibrosis, endophthalmitis, erythema elevatum et diutinum, erythroblastosis fetalis, eosinophilic fasciitis, Shulman's syndrome, Felty's syndrome, filariasis, cyclitis such as chronic cyclitis, heterochromia chronic cyclitis, iridocyclitis or Fuch's cyclitis, Henoch-Schonlein purpura, human immunodeficiency virus (HIV) infection, ECHO virus infection, cardiomyopathy, Alzheimer's disease, parvovirus infection, rubella virus infection, post-vaccination syndromes, congenital rubella infection, Epstein-Barr virus infection, mumps, Evan's syndrome, autoimmune gonadal failure, Sydenham's chorea, poststreptococcal nephritis, thromboangiitis obliterans, thyrotoxicosis, tabes dorsalis, chorioiditis, giant cell polymyalgia, endocrine ophthamopathy, chronic hypersensitivity pneumonitis, keratoconjunctivitis sicca, epidemic keratoconjunctivitis, idiopathic nephritic syndrome, minimal change nephropathy, benign familial and ischemia-reperfusion injury, retinal autoimmunity, joint inflammation, bronchitis, chronic obstructive airway disease, silicosis, aphthae, aphthous stomatitis, arteriosclerotic disorders, spermatogenesis, autoimmune hemolysis, Boeck's disease, cryoglobulinemia, Dupuytren's contracture, endophthalmia phacoanaphylactica, enteritis allergica, erythema nodosum leprosum, idiopathic facial paralysis, chronic fatigue syndrome, febris rheumatica, Hamman-Rich's disease, sensorineural hearing loss, haemoglobinuria paroxysmatica, hypogonadism, ileitis regionalis, leucopenia, mononucleosis infectiosa, transverse myelitis, primary idiopathic myxedema, nephrosis, ophthalmia symphatica orchitis granulomatosa, pancreatitis, polyradiculitis acuta, pyoderma gangrenosum, Quervain's thyroiditis, acquired spenic atrophy, infertility due to antispermatozoan antobodies, non-malignant thymoma, vitiligo, SCID and Epstein-Barr virus-associated diseases, acquired immune deficiency syndrome (AIDS), parasitic disease such as Leishmania, toxic-shock syndrome, food poisoning, disease associated with T cell infiltration, leukocyte adhesion deficiency, immune responses associated with acute and delayed hypersensitivity mediated by cytokines and T cells, leukocyte diapedesis-related disease, multiple organ injury syndrome, antigen-antibody complex mediated diseases, antiglomerular basement membrane disease, allergic neuritis, autoimmune polyendocrinopathies, oophoritis, primary myxedema, autoimmune atrophic gastritis, sympathetic ophthalmia, rheumatic diseases, mixed connective tissue disease, nephrotic syndrome, insulitis, polyendocrine failure, peripheral neuropathy, autoimmune polyglandular syndrome type I, adult-onset idiopathic hypoparathyroidism (AOIH), alopecia totalis, dilated cardiomyopathy, epidermolysis bullosa acquisita (EBA), hemochromatosis, myocarditis, nephrotic syndrome, primary sclerosing cholangitis, purulent or non-purulent sinusitis, acute or chronic sinusitis, ethmoid, frontal, maxillary or sphenoid sinusitis, eosinophilic disorder such as eosinophilia, pulmonary infiltration eosinophilia, eosinophilia-myalgia syndrome, Loffler's syndrome, chronic eosinophilic pneumonia, topical pulmonary eosinophilia, bronchopneumonic aspergillosis, aspergilloma or granulomas including eosinophils, anaphylaxis, seronegative spondyloarthritides, polyendocrine autoimmune disease, sclerosing cholangitis, sclera, episclera, chronic mucocutaneous candidiasis, Bruton's syndrome, transient hypogammaglobulinemia of infancy, Wiskott-Aldrich syndrome, ataxia telangiectasia, autoimmune disorders associated with collagen diseases, rheumatism, neurological diseases, ischemic reperfusion disorder, reduction in blood pressure response, blood vessel malfunction, angiectasis, tissue injury, cardiovascular ischemia, hyperalgesia, cerebral ischemia and disease accompanying vascularization, allergic hypersensitivity disorder, glomerulonephritides, reperfusion injury, reperfusion injury of myocardium or other tissues, dermatoses having acute inflammatory component, acute purulent meningitis or other central nervous system inflammatory disorder, ocular and orbital inflammatory disorder, granulocyte transfusion-associated syndromes, cytokine-induced toxicity, acute serious inflammation, chronic intractable inflammation, pyelitis, pneumonocirrhosis, diabetic retinopathy, diabetic large-artery disorder, endarterial hyperplasia, peptic ulcer, valvulitis and endometriosis.

Preferably, the autoimmune disease may be selected from the group consisting of type 1 diabetes, alopecia areata, anti-phospholipid antibody syndrome, rheumatoid arthritis, psoriasis or psoriatic arthritis, multiple sclerosis, systemic lupus erythematosus, inflammatory bowel disease, Addison's disease, Graves' disease, Sjogren's syndrome, Guillain-Barre syndrome, Hashimoto's thyroiditis, myasthenia gravis, inflammatory myopathy, autoimmune vasculitis, autoimmune hepatitis, hemolytic anemia, idiopathic thrombocytopenic purpura, primary biliary cirrhosis, scleroderma, vitiligo, pernicious anemia and celiac disease.

The inflammatory disease may be selected from the group consisting of arthritis, ankylosing spondylitis, reactive arthritis, Reiter's syndrome, crystal arthropathies, Lyme disease, polymyalgia rheumatica, systemic sclerosis, polymyositis, dermatomyositis, polyarteritis nodosa, Wegener's granulomatosis, Churg-Strauss syndrome, sarcoidosis, atherosclerotic vascular disease, atherosclerosis, ischemic heart disease, myocardial infarction, stroke, peripheral vascular disease, uveitis, corneal disease, iritis, iridocyclitis and cataracts.

The transplant rejection disease may be a tissue or organ transplant rejection reaction, and the tissue or organ transplant rejection reaction may be selected from rejection reactions of bone marrow transplantation, heart transplantation, corneal transplantation, intestinal transplantation, liver transplantation, lung transplantation, pancreatic transplantation, kidney transplantation and skin transplantation.

As used herein, the term "inflammatory skin disease" refers to a disease occurring in the skin by an inflammatory reaction. Skin keratinocytes are components that constitute most of the epidermal cells, and they form keratin and are involved in various inflammatory and immune responses by producing various cytokines. When skin keratinocytes are exposed to environmental and physiological stress, an inflammatory response occurs, and by this, various types of inflammatory cytokines such as tumor necrosis factor-α (TNF-α), interleukin-1β (IL-1β), IL-6 and chemokine (C-C motif) ligand (CCL) are secreted. These cytokines reduce the rate of the proliferation of keratinocytes in the skin and interfere with the formation of matrix in the dermal layer, thereby slowing the healing rate of damaged skin. Therefore, the proliferation of keratinocytes in inflammatory skin diseases plays an important role and is closely related to inflammatory skin diseases such as skin aging, atopic dermatitis, psoriasis and the like. Accordingly, the inflammatory skin disease may be psoriasis or atopic dermatitis, and preferably, psoriasis.

As used herein, the term "psoriasis" refers to a disease that forms various sizes of erythematous papules and plaques with clear boundaries that are covered with silvery-white scales on the skin. Histologically, it is one of the chronic inflammatory skin diseases characterized by epithelial hyperplasia, showing various clinical features and repeating exacerbation and improvement.

The preferred dosage of the pharmaceutical composition varies depending on the condition and weight of the patient, the degree of disease, the drug form, the route and duration of administration, but may be appropriately selected by those skilled in the art. In the pharmaceutical composition for treating or preventing psoriasis according to the present invention, the active ingredient may be included in any amount (effective amount) depending on the use, formulation, purpose of formulation and the like, as long as it can exhibit a therapeutic activity for psoriasis, and it will be determined within the range of 0.001 wt.% to 20.0 wt.% on the basis of the total weight of the composition. Herein, the term "effective amount" refers to the amount of an active ingredient capable of inducing a psoriasis treatment effect. Such effective amounts may be determined empirically within the ordinary ability of those skilled in the art.

As used herein, the term "treatment" may be used to include both therapeutic treatment and prophylactic treatment. In this case, prevention may be used in the sense of alleviating or reducing a pathological condition or disease of a subject. In an embodiment, the term "treatment" includes any form of application or administration for treating a disease in mammals, including humans. In addition, the term includes the meanings of inhibiting or slowing a disease or the progress of a disease; restoring or repairing damaged or missing function to partially or completely relieve the disease; stimulating inefficient processes; or alleviating serious diseases.

Herein, the term "therapeutically effective amount" or "pharmaceutically effective amount" refers to the amount of a compound or composition effective to prevent or treat a target disease, which is sufficient to treat the disease at a reasonable benefit/risk ratio applicable to medical treatment, and it means an amount that does not cause side effects. The level of the effective amount may be determined by the patient's health status, disease type, severity, drug activity, drug sensitivity, administration method, administration time, administration route and excretion rate, treatment period, factors including drugs used in combination or concurrently and factors well known in the medical field. In an embodiment, a therapeutically effective amount refers to the amount of a drug effective to treat psoriasis.

The composition of the present invention may include a pharmaceutically acceptable carrier, and may additionally include a pharmaceutically acceptable adjuvant, excipient or diluent in addition to the carrier.

As used herein, the term "pharmaceutically acceptable" refers to a composition that is physiologically acceptable and does not normally cause gastrointestinal disorders, allergic reactions such as dizziness or similar reactions when administered to humans. Examples of the carriers, excipients and diluents may include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methylhydroxybenzoate, propylhydroxybenzoate, talc, magnesium stearate and mineral oil. In addition, fillers, anti-agglomeration agents, lubricants, wetting agents, fragrances, emulsifiers and preservatives may be further included.

The pharmaceutical composition of the present invention may be formulated by using methods known in the art to enable rapid, sustained or delayed release of the active ingredient upon administration to mammals. Formulations include powders, granules, tablets, emulsions, syrups, aerosols, soft or hard gelatin capsules, sterile injectable solutions and sterile powder forms.

The composition of the present invention may be formulated in a suitable form together with a pharmaceutically acceptable carrier. For example, pharmaceutically acceptable carriers include carriers for parenteral administration such as water, suitable oils, saline, aqueous glucose, glycol and the like, and may further include stabilizers and preservatives. Suitable stabilizers include antioxidants such as sodium bisulfite, sodium sulfite or ascorbic acid. Suitable preservatives include benzalkonium chloride, methyl- or propyl-paraben and chlorobutanol. In addition, the composition according to the present invention may appropriately include a suspending agent, solubilizer, stabilizer, isotonic agent, preservative, adsorption inhibitor, surfactant, diluent, excipient, pH adjuster, analgesic agent, buffer, antioxidant and the like if necessary depending on the administration method or formulation. Pharmaceutically acceptable carriers and agents suitable for the present invention, including those exemplified above, are described in detail in the literature [Remington's Pharmaceutical Sciences, latest edition].

The composition of the present invention may be sterilized according to commonly known sterilization techniques. The composition may include pharmaceutically acceptable auxiliary substances and adjuvants, toxicity control agents and analogs thereof, which are required to control physiological conditions such as pH control, and for example, there are sodium acetate, sodium chloride, potassium chloride, calcium chloride, sodium lactate and the like. The concentration of the fusion protein in such formulations may vary widely, and for example, it may be about 0.5% or less depending on the weight or generally at least about 1% to 15% or up to 20%, and depending on the particular method of administration selected, it may be selected preferentially based on body fluid volume, viscosities and the like.

A preferred dosage for the composition of the present invention may be in the range of 0.01 µg/kg to 10 g/kg per day or in the range of 0.01 mg/kg to 1 g/kg, depending on the patient's condition, body weight, gender, age, patient's severity and administration route. Administration may be performed once or divided into several times a day. Such dosages should not be construed as limiting the scope of the invention in any respect.

Subjects to which the composition of the present invention can be applied (prescribed) are mammals and humans, and in particular, preferably, humans. The administration route, administration dosage and administration frequency of the fusion protein or fusion protein dimer may be administered to a subject by various methods and amounts depending on the patient's condition and presence or absence of side effects, and the optimal administration method, administration dosage and administration frequency may be selected by a person skilled in the art within an appropriate range.

The composition of the present invention may be administered by any route. The composition of the present invention may be provided to an animal either directly (e.g., topically by injection, implantation or local administration at a tissue site) or systemically (e.g., parenterally or orally) by any suitable means. When the composition of the present invention is provided parenterally such as intravenous, subcutaneous, ophthalmic, intraperitoneal, intramuscular, oral, rectal, intraorbital, intracerebral, intracranial, intraspinal, intraventricular, intrathecal, intracisternal, intracapsular, intranasal or aerosol administration, the composition is preferably aqueous or it is preferable to include portions of physiologically applicable bodily fluid suspensions or solutions. Accordingly, since the carrier or vehicle is physiologically acceptable, it may be added to the composition and delivered to the patient, without adversely affecting the patient's electrolyte and/or volumetric balance. Therefore, as a body fluid medium for the preparation, it may generally include a physiological saline.

A DNA construct (or gene construct) including a nucleic acid encoding the fusion protein of the present invention may be used as part of a gene therapy protocol carrying a nucleic acid encoding the fusion protein construct.

In the present invention, an expression vector for infecting and expressing the fusion protein *in vivo* in a specific cell type in order to reconstitute or supplement the desired function of the fusion protein may be administered together with any biologically effective carrier, and examples thereof include any formulations or compositions capable of efficiently delivering a gene encoding a desired fusion protein or a fusion protein thereof to cells *in vivo.*

For gene therapy using a nucleic acid encoding the fusion protein, the gene of interest may be inserted into a viral vector including a recombinant retrovirus, an adenovirus, an adeno-associated virus and herpes simplex virus-1, or a recombinant bacterial plasmid or a recombinant eukaryotic plasmid.

The dosage of the nucleic acid encoding the fusion protein of the present invention is in the range of 0.1 mg to 100 mg in humans, preferably, 1 mg to 10 mg, and more preferably, 2 mg to 10 mg. The optimal amount and dosage form may be determined by routine experimentation within the level of ordinary skill in the art.

The unit dose of the fusion protein of the present invention is 0.1 mg/kg to 1,500 mg/kg in humans, preferably, 1 mg/kg to 100 mg/kg, and more preferably, 5 mg/kg to 20 mg/kg. The unit dose may vary depending on the disease to be treated and the presence or absence of side effects. However, the optimal dosage may be determined using routine experimentation. Administration of the fusion protein may be by periodic bolus injections or by continuous intravenous, subcutaneous or intraperitoneal administration from an external reservoir (e.g., intravenous bag) or the inside (e.g., bioerodable implant).

The composition of the present invention may be administered in combination with other drugs or physiologically active substances having a prophylactic or therapeutic effect on the disease to be prevented or treated, or may be formulated in the form of a combination formulation with such other drugs.

The method for preventing or treating a disease using the fusion protein or composition of the present invention may include administering another drug or physiologically active substance having a prophylactic or therapeutic effect in combination with the fusion protein or composition of the present invention, and the route of concurrent administration, administration timing and administration dosage may be determined according to the type of disease, the disease state of the patient, the purpose of treatment or prevention and other drugs or physiologically active substances used in combination.

In addition, the present invention provides the use of a fusion protein including PD-L1 protein and a modified immunoglobulin Fc region for producing a pharmaceutical preparation having an effect of preventing or treating immune disease.

The present invention also provides a method for preventing or treating immune disease, including administering a fusion protein including PD-L1 protein and a modified immunoglobulin Fc region, and a pharmaceutically acceptable carrier to a subj ect.

The therapeutically effective amount is preferably applied differently depending on the specific composition including the type and extent of the response to be achieved and whether other agents are used as necessary, the subject's age, weight, general health status, gender and diet, the administration time, administration route, and secretion rate of the composition, the treatment period and drugs used together or concurrently with a specific composition and similar factors well known in the pharmaceutical field. Therefore, it is preferable to determine the effective amount of the composition suitable for the purpose of the present invention in consideration of the aforementioned factors.

The subject is applicable to any mammals, and the mammals include not only humans and primates, but also domestic animals such as cattle, pigs, sheep, horses, dogs and cats.

### [Modes of the Invention]

Hereinafter, the present invention will be described in more detail through examples. These examples are for describing the present invention in more detail, and the scope of the present invention is not limited to these examples.

### Example 1. Preparation of gene construct for production of fusion protein including PD-L1 protein and modified immunoglobulin Fc region

A gene construct was prepared for producing a fusion protein in which human programmed cell death-ligand 1 (PD-L1) protein and a modified immunoglobulin Fc domain were fused. Specifically, for the human PD-L1 gene, a known amino acid sequence (Accession Number: Q9NZQ7) was used, and the gene construct including the extracellular domain (19 to 239 aa) of the PD-L1 protein was prepared by TOP Gene Technologies. (Canada, Quebec). Human PD-L1 protein was prepared to be fused to the N-terminus of the modified immunoglobulin Fc region. The modified immunoglobulin Fc domain (SEQ ID NO: 11) is a hybrid type of human IgD Fc and human IgG4 Fc, and is characterized by including an IgG1 hinge region (SEQ ID NO: 16) composed of 8 amino acids.

The fusion protein of the present invention was prepared such that the PD-L 1 protein was linked to the modified immunoglobulin Fc region by a peptide linker. In the present invention, in order to maintain flexibility while inducing dimerization, a fusion protein (hereinafter, "PD-L1-hyFc21" or "PD-L1-hyFc21 fusion protein", SEQ ID NO: 12 or 13) was prepared, which included human PD-L1 protein and a modified immunoglobulin Fc region linked by a GS linker (SEQ ID NO: 10) consisting of a 7 amino acid sequence. As a control, a fusion protein including an immunoglobulin Fc region including the IgD hinge region of SEQ ID NO: 14 (hereinafter, "PD-L1-hyFc5" or "PD-L1-hyFc5 fusion protein", SEQ ID NO: 15) was used.

A recombinant expression vector was prepared using the gene construct including the nucleotide encoding the fusion protein. The prepared recombinant expression vector was transformed using a gene transfer method (NeonTM kit, 10 µL, Invitrogen Cat. MPK1096) in which DNA was introduced into the cell by suspending a DNA solution in the CHO DG44 cell line and passing a pulse of high direct current. Afterwards, the amplification step of HT selection (HT supplement, Invitrogen, 11067-030) and MTX (Methotrexate, Sigma, M8407) was performed, and cell passage was performed such that only cells with a high expression rate were selected. Cells were passaged in an amount of 0.4 × 10⁶ cells/mL every 3 days, and the number and viability of cells were measured using a cell counting device (Vi-cell, Beckman coulter). HT selection is a selection method in which only transformed cells survive by removing HT from the media, and MTX amplification is a method of amplifying genes by placing MTX at a selected concentration in the passage medium. The selected cell pool was subjected to single cell cloning using the limiting dilution cloning method. Briefly, cells were aliquoted to 1 cell/well in a 96-well plate, and cell images were stored on days 0, 7 and 14 using a CSI device (clone selection imager, Molecular Devices) to trace back clones derived from one cell. The productivity of the selected single cell-derived cell line was confirmed by using Fc ELISA (Human IgG ELISA Quantitation Set, Bethyl, E80-104). The finally selected 5 to 6 clones were subjected to batch culture and long-term stability evaluation, and clones whose stability was confirmed were prepared as a research cell bank (RCB).

### Example 2. Securing of PD-Ll-hyFc21 or PD-Ll-hyFc5 fusion protein

In order to mass produce the PD-L1-hyFc21 or PD-L1-hyFcS fusion protein, the target protein was isolated from the cell culture medium produced from the PD-L1-hyFcS or PD-L1-hyFc21 suspension cell line obtained in Example 1 and purified.

In order to secure a large amount of the PD-L1-hyFc21 or PD-L1-hyFcS fusion protein, a cell line expressing the PD-L1-hyFc21 or PD-L1-hyFcS fusion protein was cultured for 20 days in Glass bioreactor 15L by the same fed-batch culture method to produce the PD-L1-hyFc21 or PD-L1-hyFcS fusion protein. During fed-batch culture, the cell viability of the cell line expressing the fusion protein was measured, and size exclusion chromatography (SE-HPLC) was performed to confirm the final expression level and purity of the target protein.

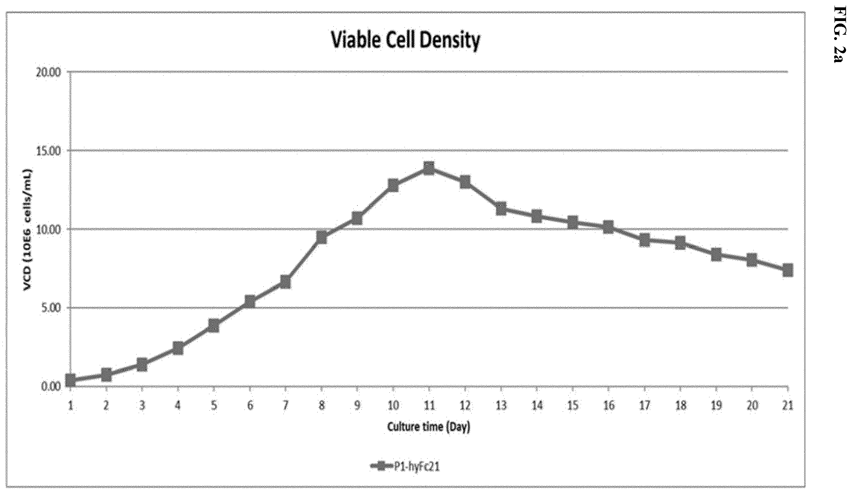

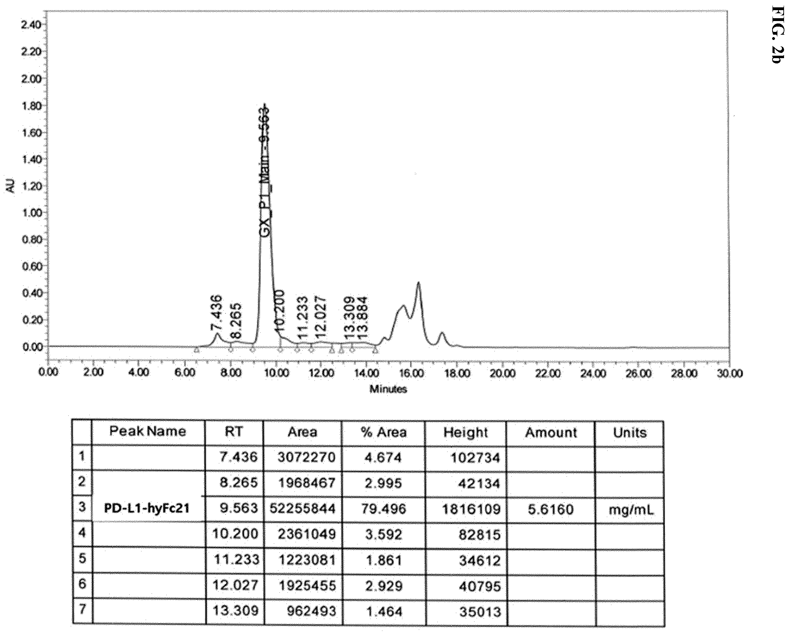

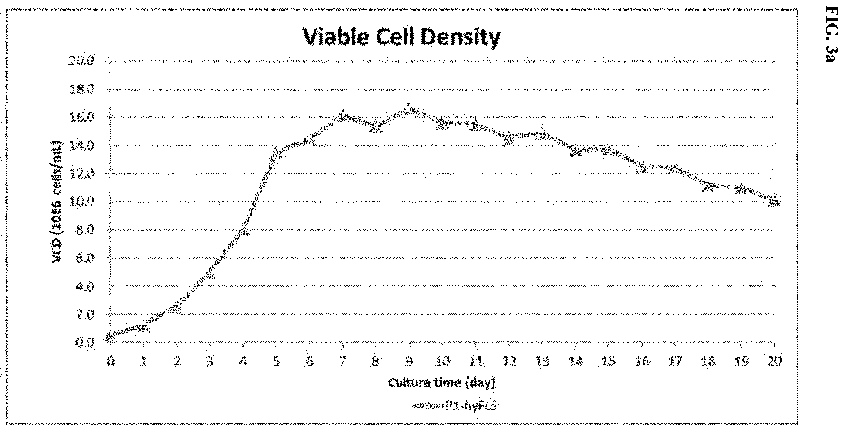

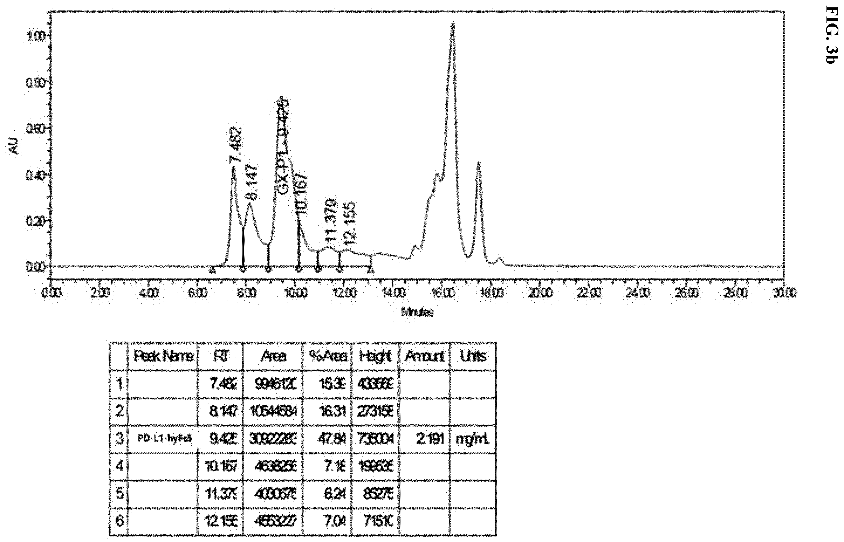

As a result of confirming the maximum cell concentration and the final expression level of the fusion protein of the expression cell line over time, the expression cell line of the PD-L1-hyFc21 fusion protein was confirmed to have a maximum cell concentration of 13.9 × 10⁶ cells/mL over time (FIG. 2a), and the expression level of the finally recovered fusion protein was confirmed to be 5.6 g/L (FIG. 2b). In comparison, the expression cell line of the PD-L1-hyFcS fusion protein was confirmed to have a maximum cell concentration over time of 16.7 × 10⁶ cells/mL (FIG. 3a), and the expression level of the finally recovered fusion protein was confirmed to be 2.2 g/L (FIG. 3b).

In addition, as a result of confirming the purity of the fusion protein, it was confirmed that the purity of the PD-L1-hyFc21 fusion protein was 79.5%, and it was confirmed to include 7.7% of high molecular weight impurities (HMW) and 9.8% of low molecular weight impurities (LMW) (Table 1). In comparison, the purity of the PD-L1-hyFcS fusion protein was 47.8%, and it was confirmed to include 31.7% of high molecular weight impurities (HMW) and 20.5% of low molecular weight impurities (LMW) (Table 1).

**[Table 1]**

| Area (%) | PD-L1-hyFc21 | PD-L1-hyFc5 |
|---|---|---|
| HMW (%) | 7.7 | 31.7 |
| Main (%) | 79.5 | 47.8 |
| LMW (%) | 9.8 | 20.5 |

| | | |
|---|---|---|
| *HMW: high molecular weight impurity; Main: target protein; and LMW: low molecular weight impurity. | | |

Afterwards, in order to obtain high-purity PD-L1-hyFc21 or PD-L1-hyFcS fusion protein, the following three-step purification process was performed: Culture medium => Protein A affinity chromatography => Anion exchange chromatography 1 => Anion exchange chromatography 2 => Ultrafiltration/diafiltration.

The obtained target protein was confirmed for purity and yield through size exclusion chromatography (SE-HPLC) analysis. As a result, it was confirmed that the purity of the PD-L1-hyFc21 fusion protein was 97.3% and the yield was 30.2%. In comparison, it was confirmed that the purity of the PD-L1-hyFc5 fusion protein was 93.2% and the yield was 5.1% (Table 2).

**[Table 2]**

| Process | PD-L1-hyFc21 | | PD-L1-hyFc5 | |
|---|---|---|---|---|
| Name of test solution | Purity (SE-HPLC) | Yield (%) | Purity (SE-HPLC) | Yield (%) |
| Culture medium | 79.5 | 100.0 | 47.8 | 100.0 |
| Final purification | 97.3 | 30.2 | 93.2 | 5.1 |

From the above results, it was confirmed that the PD-L1-hyFc21 fusion protein showed a production amount which was about 2.5 times higher than that of the PD-L1-hyFcS fusion protein, and the purity of the protein was also high.

### Example 3. Characterization analysis of PD-L1-hyFc21 or PD-Ll-hyFc5 fusion protein

### 3.1. Polyacrylamide gel electrophoresis (sodium dodecyl sulfate-polyacrylamide gel electrophoresis, SDS-PAGE)

In order to confirm the molecular weight of the purified PD-L 1-hyFc21 or PD-L1-hyFc5 fusion protein, sodium dodecyl sulfate polyacrylamide gel electrophoresis (SDS-PAGE) was performed. Briefly, the fusion protein was diluted with deionized water, mixed with NuPAGE^{™}LDS Sample Buffer (Thermo Fisher Scientific) and loaded on 4-12% Bis-Tris gel (Invitrogen) to 3 µg/well to perform electrophoresis. After electrophoresis, the gel was stained by the Coomassie staining method.

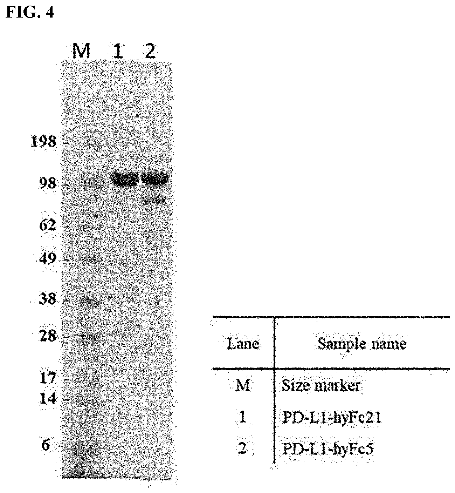

As a result, both of the purified PD-L1-hyFc21 and PD-L1-hyFcS fusion proteins were confirmed at a size marker of 98 kDa under non-reducing conditions (FIG. 4). However, the purified PD-L1-hyFcS fusion protein included a cleaved form of low-molecular impurities.

### 3.2. Size-exclusion chromatography (SE-HPLC)

In order to analyze the main peak and impurity peaks such as dimer, multimer or truncated abnormal peptides in the purified PD-L1-hyFc21 or PD-L1-hyFcS fusion protein, the size-exclusion chromatography (SE-HPLC) was performed. Briefly, after the fusion protein was diluted and prepared with formulation buffer to 1.0 mg/mL, the area ratio (% area) of the main peak of the fusion protein among the separated peaks was analyzed by using a gel filtration chromatography column (TOSOH TSK-GEL G3000SWxL column, 7.8 mm × 300 mm).

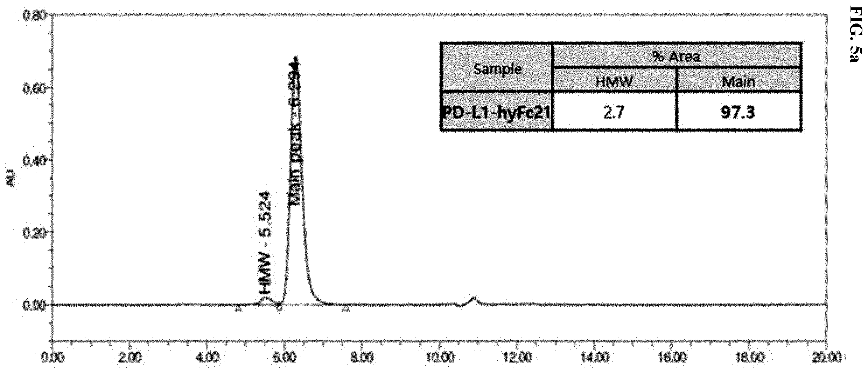

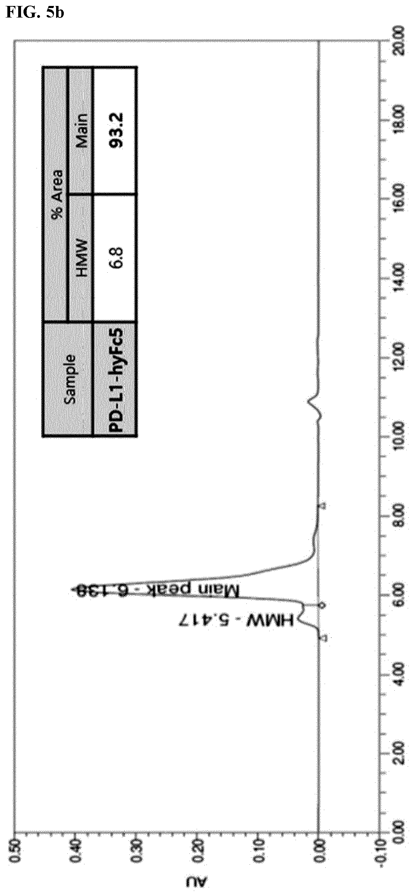

As a result, it was confirmed that the purity of the PD-L1-hyFc21 fusion protein was 97.3% (FIG. 5a), whereas the purity of the PD-L1-hyFcS fusion protein was confirmed to be 93.2% (FIG. 5b).

### 3.3. Isoelectric focusing (IEF)

In order to confirm the charge variants and distribution of the purified PD-L1-hyFc5 and PD-L1-hyFc21 proteins, isoelectric focusing (IEF) was performed. Isoelectric point electrophoresis is an electrophoresis method that analyzes separated proteins using the pI value of the protein, and the pI value refers to the pH value at which a charged protein becomes electrically neutral, which is called the isoelectric point. In isoelectric point electrophoresis, the protein that has reached the isoelectric point does not move anymore and stays on the gel and is separated. Briefly, in the present invention, the fusion protein was separated according to the pI value using a pH 3 to 10 isoelectric point gel (Invitrogen), and the gel after electrophoresis was fixed with a 12% trichloroacetic acid (TCA) solution and then stained by the Coomassie staining method.

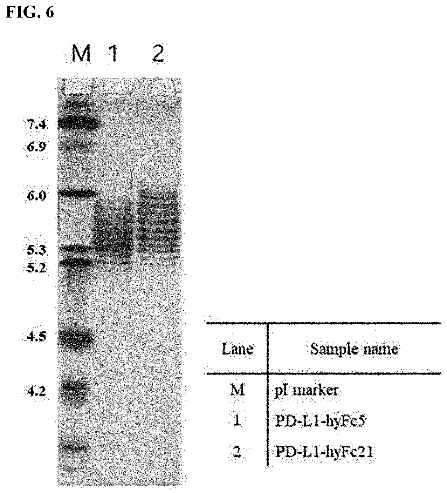

As a result, both of the PD-L1-hyFc21 and PD-L1-hyFc5 fusion proteins were confirmed to have pI values in the range of 5.2 to 6.0 (FIG. 6).

### 3.4. Differential scanning fluorimetry (DSF)

In order to analyze the heat stability of the purified PD-L1-hyFc21 or PD-L1-hyFc5 fusion protein, the experiment was performed using the PROTEOSTAT^{®} Thermal Shift Stability Assay Kit. Since the assay kit contains a fluorescent dye that detects protein aggregation, it is possible to confirm the temperature at which a large amount of protein aggregates under heat stress conditions. The aggregation temperature (tagg) is an indicator of protein stability, and it can confirm the stability of the protein structure.

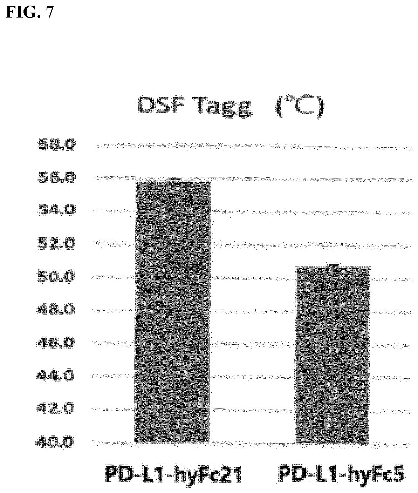

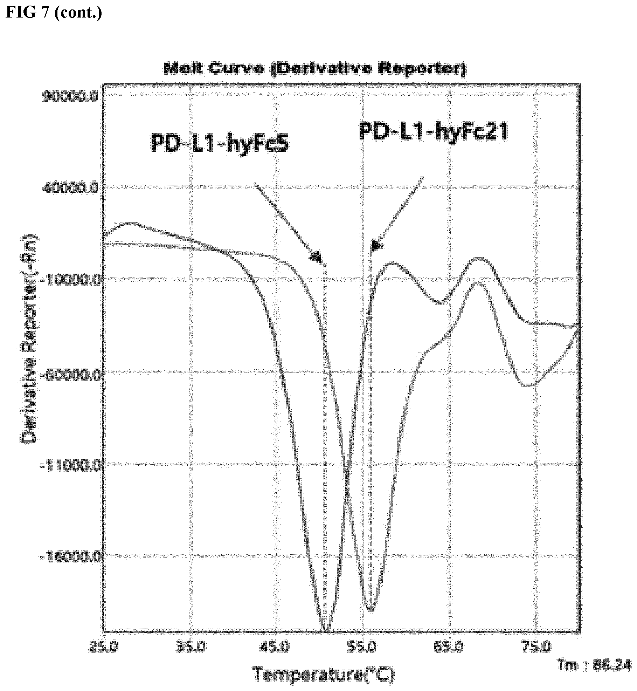

As a result, the aggregation temperature of the PD-L1-hyFc21 fusion protein was confirmed to be 55.8 °C, whereas the aggregation temperature of the PD-L1-hyFc5 fusion protein was confirmed to be 50.7 °C (FIG. 7). As a result, it was confirmed that the heat stability of the PD-L1-hyFc21 fusion protein was structurally more excellent compared to PD-L1-hyFc5.

### Example 4. In vitro activity analysis of PD-L1-hyFc21 or PD-L1-hyFc5 fusion protein

### 4.1. Binding affinity analysis for PD-1 using Jurkat (SHP-1) cell line expressing PD-1

The binding affinity of the PD-L1-hyFc21 or PD- L 1- hy F c5 fusion protein to PD-1 was analyzed using a Jurkat (SHP-1) cell line expressing PD-1. Briefly, Jurkat cells (PathHunter^{®} Jurkat PD-1 (SHP-1), DiscoverX) were aliquoted in a 96-well plate and stabilized in a 37 °C 5% CO₂ incubator for 2 hours. Afterwards, the PD-L1-hyFc21 or PD-L1-hyFc5 fusion protein was added so as to become 75 and 600 nM, respectively, and then reacted in a 37 °C 5% CO₂ incubator for 1 hour. After completion of the reaction, a reagent (PathHunter^{®} Bioassay Detection Kit, DiscoverX) for detecting SHP-1 expressed by the PD-1:PD-L1 signaling response was added to measure the luminescence of SHP-1.

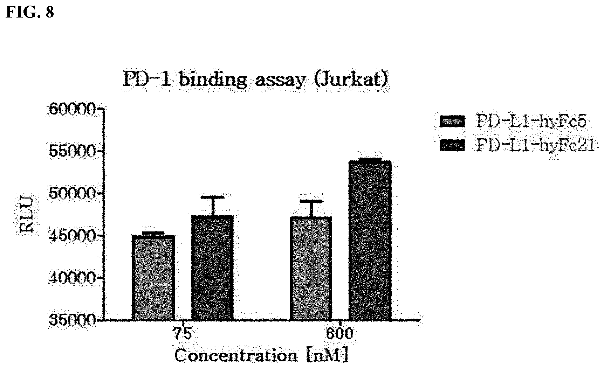

As a result, the group treated with the PD-L1-hyFc21 fusion protein showed a higher degree of luminescence of SHP-1 at both concentrations of 75 and 600 nM, compared to the group treated with the PD-L1-hyFc5 fusion protein (FIG. 8). Accordingly, it was confirmed that the PD-L1-hyFc21 fusion protein had a higher binding affinity to PD-1 than the PD-L1-hyFc5 fusion protein.

### 4.2. Analysis of inhibitory capacity of mixed lymphocyte reaction

The inhibitory capacity of the mixed lymphocyte response by the PD-L1-hyFc21 or PD-L1-hyFc5 fusion protein was analyzed. Briefly, donor and recipient peripheral blood mononuclear cells (PBMCs) were prepared and subjected to CTV (CellTrace^{™} Violet) and CTR (CellTrace^{™} Far Red) staining for intracellular proteins. Each of the donor and recipient PBMCs was placed as response cells and stimulator cells, mixed at a ratio of 1:1 and placed in a 96-well U-bottom plate, and after 0.5 µM of the PD-L1-hyFc21 or PD-L1-hyFc5 fusion protein was added, it was reacted in a 37 °C 5% CO₂ incubator for 5 days. By analyzing the degree of decrease in the fluorescence of CTV (CellTrace^{™} Violet) stained on the response cells in the cells in which the reaction had been completed, the degrees of inhibition of the mixed lymphocyte reaction by the PD-L1-hyFc21 or PD-L1-hyFc5 fusion protein were compared.

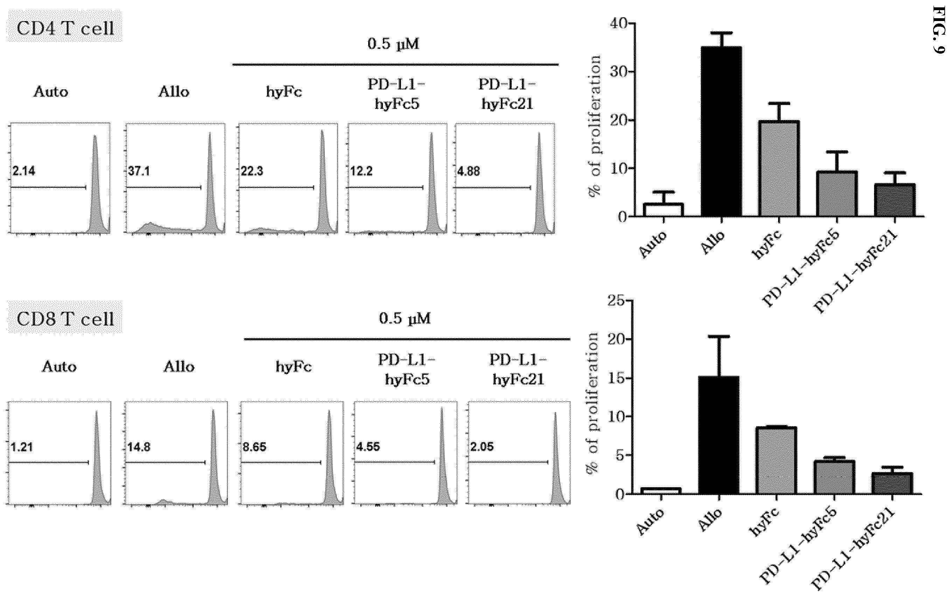

As a result, the proliferation of CD4 T cells and CD8 T cells of the activated response cells by the mixed lymphocyte reaction was reduced in the group treated with the PD-L1-hyFc21 or PD-L1-hyFcS fusion protein, compared to the control group (Allo, hyFc treated group) (FIG. 9). In particular, it was confirmed that the proliferations of CD4 T cells and CD8 T cells of the activated response cells were significantly reduced in the group treated with the PD-L1-hyFc21 fusion protein at the same concentration condition, compared to the group treated with the PD-L1-hyFcS fusion protein (FIG. 9).

### 4.3. Analysis of proliferation inhibition and cytokine production inhibition of activated human T cells

The inhibition capacities of the proliferation of human T cells by the PD-L1-hyFc21 or PD-L1-hyFcS fusion protein were compared under T cell activation conditions using human PBMCs. Briefly, the PD-L1-hyFc21 or PD-L1-hyFc5 fusion protein together with anti-CD3 antibody was treated in a 96-well plate and coated at 4 °C for one day. After CD4 T cells were isolated from PBMCs of normal people using microbeads (MACS), CTV (CellTrace^{™} Violet, 2.5 µM) staining for intracellular proteins was performed on the isolated CD4 T cells. After the coated plate was washed with PBS, CD4 T cells stained with CTV were placed in a test plate and cultured in a 37 °C 5% CO₂ incubator for 3 days. After 4 days, the cells from the test plate were harvested, and the degree of decrease in CTV fluorescence was analyzed using flow-cytometry.

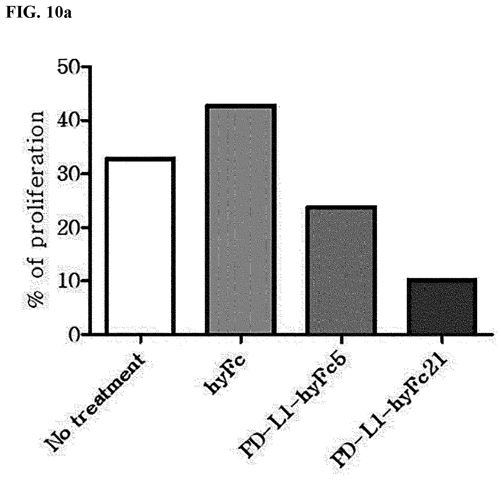

As a result, it was confirmed that the proliferation of activated human T cells was reduced in the group treated with the PD-L1-hyFc21 or PD-L1-hyFcS fusion protein, compared to the control group (no treatment or hyFc treatment group) (FIG. 10a). In particular, it was confirmed that the proliferation of activated human T cells was significantly inhibited by about 2 times in the group treated with the PD-L1-hyFc21 fusion protein, compared to the group treated with the PD-L1-hyFcS fusion protein (FIG. 10a).

In addition, the inhibition capacities of the cytokine expression of human T cells by the PD-L1-hyFc21 or PD-L1-hyFcS fusion protein were compared under T cell activation conditions using human PBMCs. Briefly, the PD-L1-hyFc21 or PD-L1-hyFcS fusion protein together with anti-CD3 antibody was diluted to be treated at 0.5 µM and coated in a 48-well plate at 4 °C for one day. CD4 T cells were isolated from normal human PBMCs using microbeads (Miltenyi Biotech). CD4 T cells were isolated from normal human PBMCs using microbeads (MACS). After the coated plate was washed with PBS, the isolated CD4 T cells were placed in a test plate and cultured in a 37 °C 5% CO₂ incubator for 3 days. Anti-IFN-γ antibody (DuoSet Human IFN-gamma ELISA set, R&D system) was placed in a test plate and coated at room temperature. After washing the coated test plate with PBS (PBST), in which 0.05% Tween-20 was added, 3 times, PBS in which 1% BSA was added was blocked in the test plate at room temperature for 2 hours. The culture medium was transferred to the CD4 cells on the test plate and reacted at room temperature for 2 hours. After washing 5 times with PBST, the detection antibody was added and reacted for 2 hours at room temperature, followed by washing 5 times with PBST. After adding streptavidin-HRP to the washed test plate, it was reacted at room temperature for 20 minutes. Afterwards, it was washed 5 times with PBST, and after the TMB substrate was added, sulfuric acid (H₂SO₄) at a concentration of 2N was added to measure the absorbance at 450 nm. The ability of the PD-L1-hyFc21 or PD-L1-hyFcS fusion protein to inhibit IFN-γ production by activated CD4 T cells was analyzed using the measured absorbance values.

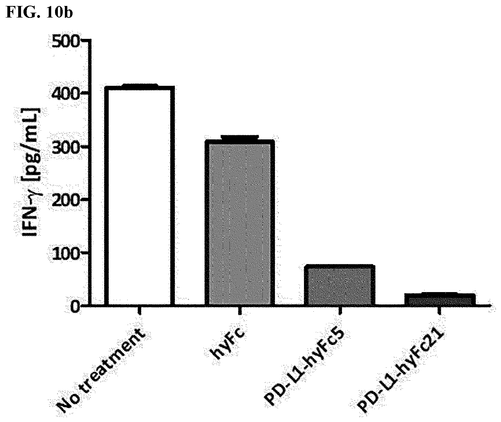

As a result, it was confirmed that the amount of IFN-γ produced in activated human CD4 T cells was significantly reduced in the group treated with the PD-L1-hyFc21 or PD-L1-hyFcS fusion protein, compared to the control group (no treatment or hyFc treatment group) (FIG. 10b). In particular, the amount of IFN-γ generated from activated human CD4 T cells was significantly inhibited in the group treated with the PD-L1-hyFc21 fusion protein by about 3 times, compared to the group treated with the PD-L1-hyFcS fusion protein (FIG. 10b).

### 4.4. Analysis of cytokine production inhibition of activated mouse T cells

The inhibition capacities of the cytokine expression of mouse T cells by the PD-L1-hyFc21 or PD-L1-hyFcS fusion protein were compared under the mouse T cell activation conditions. Briefly, the PD-L1-hyFc21 or PD-L1-hyFcS fusion protein (0.5 µM) together with 5 µg/mL of anti-CD3 antibody was placed in a test plate and coated at 4 °C for one day. CD4 T cells were isolated from lymphocytes isolated from the lymph nodes of mice using microbeads (Miltenyi Biotech). After washing the coated plate with PBS, the isolated CD4 T cells were added and cultured in a 37° C 5% CO₂ incubator at for 3 days. Anti-IL-2 antibody (Mouse IL-2 ELISA MAX^{™} Deluxe, Biolegend) and anti-IFN-γ antibody (Mouse IFNgamma ELISA MAX^{™} Deluxe, Biolegend) were placed in a new test plate and coated at room temperature. The test plate coated the previous day was washed 4 times with washing buffer, and then, assay diluent A was added and blocked at room temperature for 1 hour. After washing the test plate 4 times with washing buffer again, the CD4 T cell culture medium was transferred and reacted at room temperature for 2 hours. After washing 4 times with washing buffer, the detection antibody was added and reacted with shaking at room temperature for 1 hour, and washed 4 times with washing buffer again. After adding streptavidin-HRP to the washed test plate, it was reacted at room temperature for 30 minutes. After washing with washing buffer 5 times, the TMB substrate was added, and it was reacted for 30 minutes at room temperature to block light. Absorbance was measured at 450 nm within 15 minutes by adding a stop solution. The measured absorbance values were used to calculate the concentrations of IL-2 and IFN-γ, and the ability of the PD-L1-hyFc21 or PD-L 1-hyFcS fusion protein to inhibit the production of IL-2 and IFN-γ produced by activated CD4 T cells was analyzed.

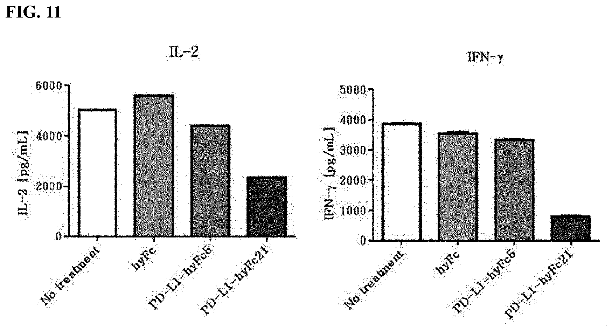

As a result, it was confirmed that the amounts of IL-2 and IFN-γ produced in the activated mouse CD4 T cells were significantly reduced in the group treated with the PD-L1-hyFc21 or PD-L1-hyFcS fusion protein, compared to the control group (no treatment or hyFc treatment group) (FIG. 11). In particular, it was confirmed that the amount of IL-2 generated from activated human CD4 T cells was significantly inhibited in the group treated with the PD-L1-hyFc21 fusion protein by about 1.8 times, compared to the group treated with the PD-L1-hyFc5 fusion protein, and the amount of IFN-γ was significantly inhibited by about 4 times (FIG. 11).

### Example 5. In vivo activity analysis of PD-L1-hyFc21 fusion protein

### 5.1. Efficacy evaluation of PD-L1-hyFc21 fusion protein in imiquimod (IMQ)-induced psoriasis mouse model

When psoriasis develops, dendritic cells are activated by toll like receptor 7/8 (TLR7/8), and as a ligand of TLR7 and TLR8, imiquimod (IMQ) is known to cause an inflammatory response like psoriasis, when it is repeatedly applied to mouse skin. Imiquimod (IMQ) is involved not only in dendritic cells but also in T cells, which are adaptive immune cells, and among them, gamma delta T cells (γδT17) that secrete IL-17 (interleukin-17) are preferentially involved in psoriatic inflammation. Therefore, the IMQ-induced psoriasis mouse model has a phenotype similar to that of psoriasis patients, and it can be used as a suitable model for the development of psoriasis therapeutics.

Briefly, in order to induce psoriasis in a mouse animal model, IMQ cream was applied to both ears of mice daily at 20 mg/mouse/day for 6 days. The ear thickness of the mice in each group was measured using a caliper every day for 7 days from day 0, when the IMQ cream was applied, to day 6, the end date of the test, and the changes in the ear thickness of the mice of each group were compared based on the ear thickness of mice not applied with IMQ cream (no treatment). The PD-L1-hyFc21 fusion protein was administered subcutaneously or intravenously on days 0, 1, 3 and 5. Alternatively, the anti-IL12p40 antibody was administered intravenously (iv) once on day 2 of the test. The control groups and the experimental groups are shown in Table 3 below.

**[Table 3]**

| Group | | Inducer | Dose | N | Route | Volume |
|---|---|---|---|---|---|---|
| Control | 1 | Vehicle | - | 5 | Subcutaneous | 3 mL/kg |
| | 2 | IMQ | - | 5 | | 3 mL/kg |
| PD-L1-hyFc21 | 3 | IMQ | 3 mg/kg | 5 | Subcutaneous | 3 mL/kg |
| | 4 | IMQ | 10 mg/kg | 5 | | 3 mL/kg |
| | 5 | IMQ | 30 mg/kg | 5 | | 3 mL/kg |
| | 6 | IMQ | 3 mg/kg | 5 | Intravenous | 5 mL/kg |
| | 7 | IMQ | 10 mg/kg | 5 | | 5 mL/kg |
| | 8 | IMQ | 30 mg/kg | 5 | | 5 mL/kg |

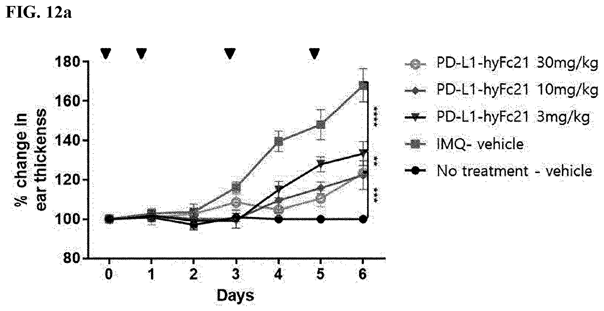

As a result, it was confirmed that the ear thicknesses increased by about 68% on day 6, the end date of the test, in the IMQ-treated group (IMG-vehicle) compared to the control group not treated with IMQ (no treatment-vehicle) (FIG. 12a). On the other hand, differences were observed in the ear thicknesses of mice from day 4 in the groups subcutaneously administered with 3, 10 and 30 mg/kg of the PD-L1-hyFc21 fusion protein (FIG. 12a). In particular, the ear thicknesses were suppressed by 51% on day 6, the end date of the test, in the group subcutaneously administered with the lowest dose of 3 mg/kg of the PD-L1-hyFc21 fusion protein, compared to the group treated with IMQ (IMG-vehicle), and the groups subcutaneously administered with the medium and high doses of 10 mg/kg and 30 mg/kg of the PD-L1-hyFc21 fusion protein showed 67% and 66% inhibition, respectively, compared to the IMQ-treated group (IMG-vehicle) (FIG. 12a). In addition, there was a significant difference according to the dose between the groups administered with 10 mg/kg or 30 mg/kg and the group administered with a low dose of 3 mg/kg, but there was no significant difference between the group administered with 10 mg/kg and the group administered with 30 mg/kg.

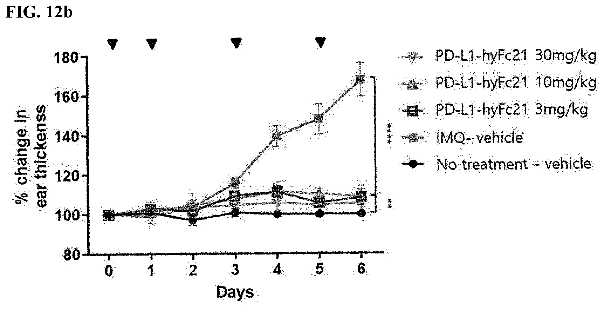

In addition, it was confirmed that the ear thickness of the mice was significantly inhibited by about 87 to 92% until day 6, the end date of the test, in all of the groups administered intravenously with 3, 10 and 30 mg/kg of the PD-L1-hyFc21 fusion protein, compared to the IMQ-treated group (IMG-vehicle) (FIG. 12b).

### 5.2. Efficacy evaluation according to subcutaneous administration of PD-L1-hyFc21 fusion protein in psoriasis mouse model transformed with doxycycline-induced Pelil gene

In order to evaluate the efficacy of the PD-L1-hyFc21 fusion protein in a psoriasis mouse model of chronic inflammation, the inventors of the present invention used a psoriasis mouse model (hereinafter, "rtTA-Peli1 psoriasis mouse model") in which long-term chronic psoriasis symptoms were induced by transforming the Peli1 (Pellino homolog 1) gene to be overexpressed according to the administration of doxycycline in addition to the imiquimod (IMQ)-induced psoriasis mouse model. As psoriasis is induced, the rtTA-Peli1 psoriasis mouse model causes the development of psoriatic lesions in the epidermis, an increase in inflammatory responses such as an increase in inflammatory cytokines, an increase in the epithelial cell layer, and an increase in the infiltration of phagocytes, dendritic cells and Th17 cells into the dermis.

Briefly, in order to induce psoriasis in mice, 4-week-old mice were allowed to drink drinking water containing 5% sucrose and 2 mg/mL of doxycycline for 6 months. Saline containing doxycycline was continuously supplied during the test period. Mice that were not transformed with the Peli gene (hereinafter, "rtTA") were used as a control group. The PD-L1-hyFc21 fusion protein was administered subcutaneously once a week for 8 weeks.

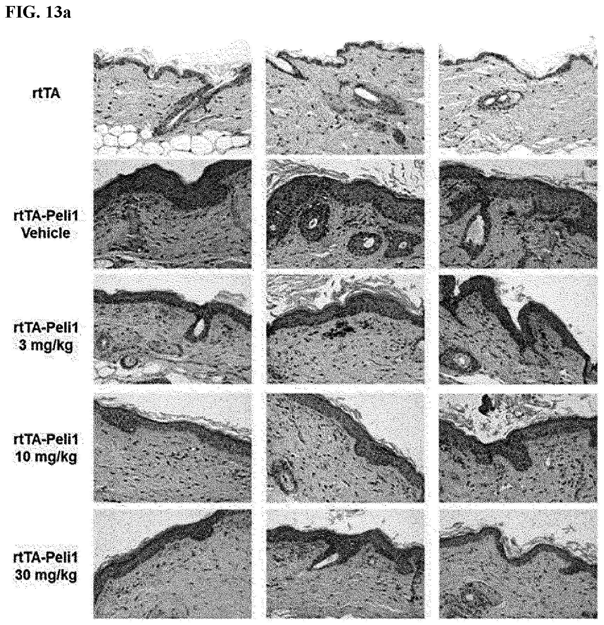

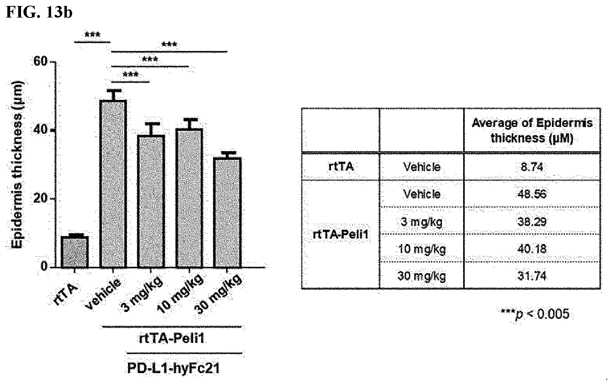

First, after subcutaneous administration of the PD-L1-hyFc21 fusion protein to the rtTA-Peli1 psoriasis mouse model, an experiment was performed to measure changes in the skin epithelial tissue and the thickness of the skin epithelial layer through H&E staining. The results of H&E staining were observed at 200X magnification, and 6 parts of each picture were arbitrarily designated and measured. As a result, the average thickness of the epithelial layer was 8.74 µm in the non-psoriasis control group (rtTA), and the average thickness of the epithelial layer was 48.56 µm in the psoriasis-induced rtTA-Peli1 group (rtTA-Peli1 Vehicle), and it was confirmed that the thickness of the epithelial layer in the rtTA-Peli 1 group (rtTA-Peli 1 Vehicle) was increased by about 5.6 times compared to the control group (FIGS. 13a and 13b). On the other hand, the average epithelial layer thicknesses were 38.29 µm, 40.18 µm and 31.74 µm in the groups subcutaneously administered with 3, 10 and 30 mg/kg of the PD-L1-hyFc21 fusion protein, respectively, and it was confirmed that there was an effect of reducing the thickness of the epithelial layer compared to the psoriasis-induced rtTA-Peli1 group (rtTA-Peli1 Vehicle) (FIGS. 13a and 13b). However, no dose-dependent effect was observed.

In addition, after subcutaneous administration of the PD-L1-hyFc21 fusion protein to the rtTA-Peli1 psoriasis mouse model, an experiment was performed to compare the degrees of infiltration of T cells and macrophages into the dermal layer through immunofluorescence staining. An anti-CD3 antibody was used for the detection of T cells, and an anti-F4/80 antibody was used for the detection of macrophages. The results were observed at 400X magnification of an immunofluorescence microscope, and the number of cells in the same area was calculated.

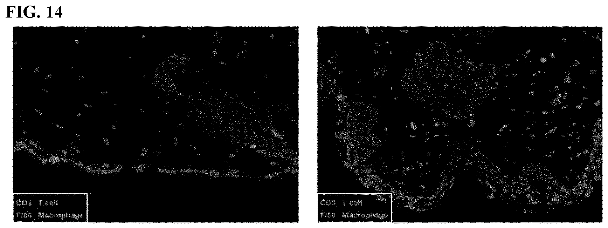

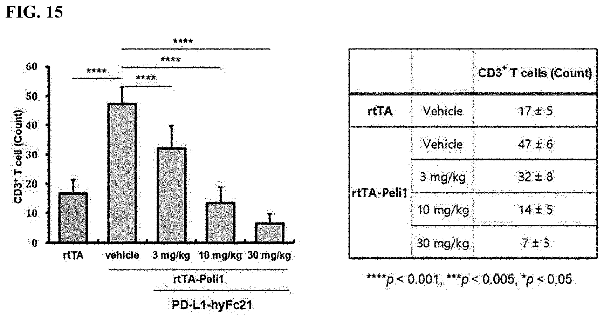

As a result, it was confirmed that T cells and macrophages hardly infiltrated into the skin tissue in the control group (rtTA) not induced with psoriasis, and it was confirmed that the numbers of T cells and macrophages infiltrated into the skin tissue were increased in the psoriasis-induced rtTA-Peli1 group (rtTA-Peli1 Vehicle) (FIG. 14). In particular, the number of T cells infiltrated into the skin tissue in the psoriasis-induced rtTA-Peli1 group (rtTA-Peli1 Vehicle) was about 47 (FIG. 15). On the other hand, the numbers of T cells infiltrated into the skin tissue were about 32, 14 and 7 in the groups subcutaneously administered with 3, 10 and 30 mg/kg of the PD-L1-hyFc21 fusion protein, respectively, and it was confirmed that the number of T cells was significantly reduced in a dose-dependent manner (FIG. 15).

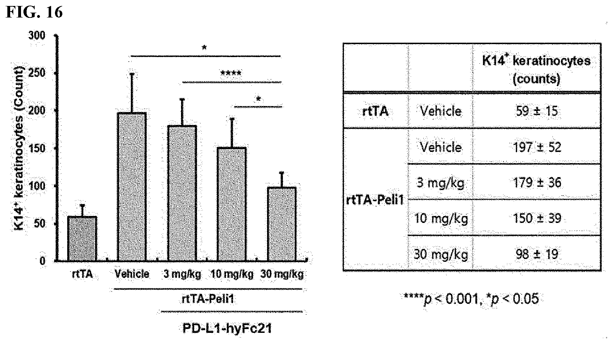

In addition, after subcutaneous administration of the PD-L1-hyFc21 fusion protein to the rtTA-Peli1 psoriasis mouse model, changes in keratinocytes in the skin tissues were analyzed by immunofluorescence staining using an anti-K14 antibody. The expression of keratin 14 (K14) appears in the basal layer responsible for proliferation, and an increase in their expression can be interpreted as an increase in the number of cells responsible for proliferation among keratinocytes. The results were compared by measuring the number of cells in the same area. As a result, the number of K14⁺ keratinocytes was 59 in the non-psoriasis-induced control group (rtTA), and the number of keratinocytes was 197 in the psoriasis-induced rtTA-Peli1 group (rtTA-Peli1 Vehicle), and thus, the number of keratinocytes in the rtTA-Peli1 group increased by about 3.3 times, compared to the control group (FIG. 16). On the other hand, the numbers of keratinocytes were about 179, 150 and 98 in the groups subcutaneously administered with 3, 10 and 30 mg/kg of the PD-L1-hyFc21 fusion protein, respectively, and it was confirmed that the number of keratinocytes decreased in a dose-dependent manner, and particularly, the number of keratinocytes was significantly reduced at a dose of 30 mg/kg (FIG. 16).

### 5.3. Efficacy evaluation according to intravenous administration of PD-L1-hyFc21 fusion protein in psoriasis mouse model transformed with doxycycline-induced Pelil gene

After intravenous administration of the PD-L1-hyFc21 fusion protein to the rtTA-Peli1 psoriasis mouse model once a week for 5 weeks, changes in the epithelial layer of the skin were analyzed as a score index, and an experiment was performed to measure the skin thickness of the abdominal region using a caliper. The score index was evaluated as follows: 0 = normal skin; 1 = keratoplasia appear; 2 = keratoplasia appear on half of back skin, or skin lesions slightly overtop the normal skin; 3 = thickness appear on whole back skin, or skin lesions significantly overtop the normal skin; 4 = skin lesions sclerosis (Biomedicine & Pharmacotherapy, Volume 110, February 2019, Pages 265-274).

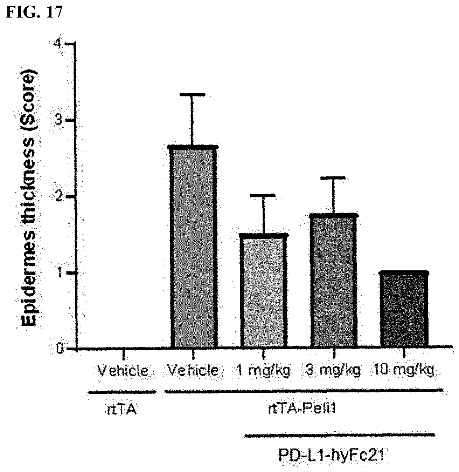

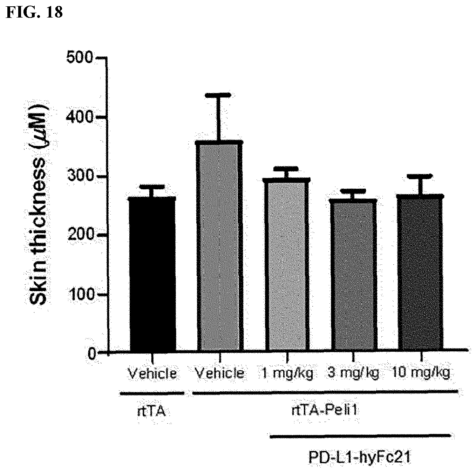

As a result, the score index increased by nearly 3 in the psoriasis-induced rtTA-Peli1 group (rtTA-Peli1 Vehicle), compared to the control group (rtTA), whereas it was confirmed that the score index was reduced in the group intravenously administered with the PD-L1-hyFc21 fusion protein, compared to the rtTA-Peli1 group (FIG. 17). In addition, similar to the score index, the skin thickness of the abdominal region increased by about 100 µm in the psoriasis-induced rtTA-Peli1 group (rtTA-Peli1 Vehicle), compared to the control group (rtTA), whereas it was confirmed that the skin thickness was reduced to a level similar to that of the control group (rtTA) in the group intravenously administered with the PD-L 1-hyFc21 fusion protein (FIG. 18). However, no dose-dependent effect was confirmed in the above results.

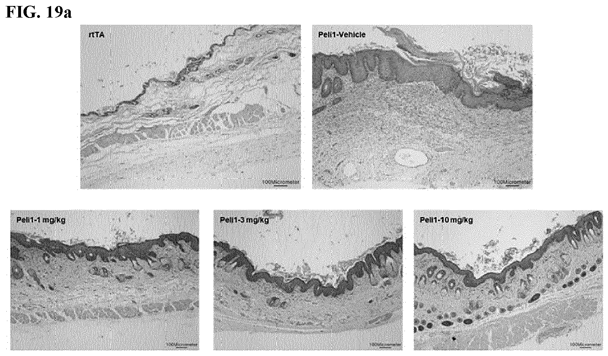

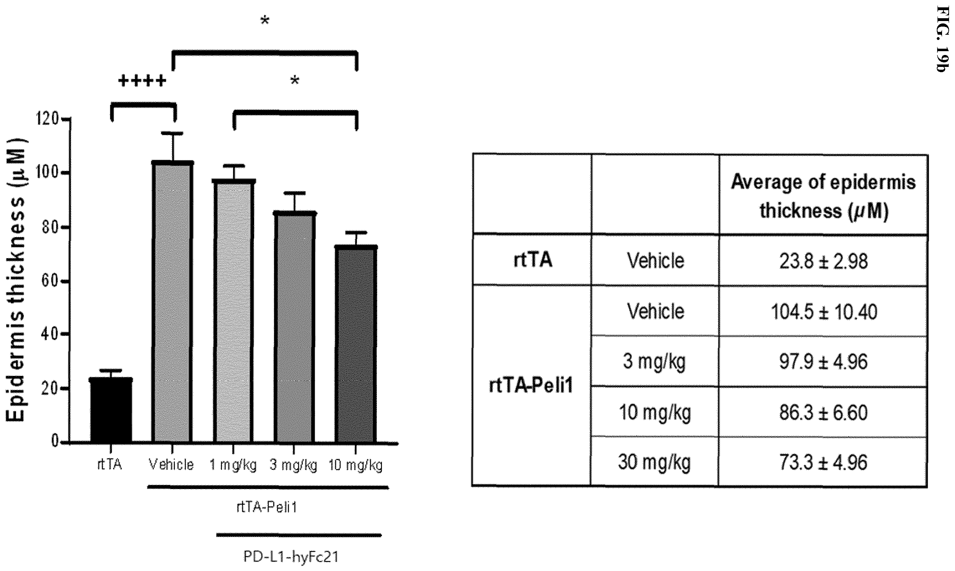

In addition, after intravenous administration of the PD-L1-hyFc21 fusion protein to the rtTA-Peli1 psoriasis mouse model, an experiment was performed to measure changes in the skin epithelial tissue and the thickness of the skin epithelial layer through H&E staining. The results of H&E staining were observed at 400X magnification of a microscope, 6 parts of the epithelial layer were arbitrarily designated, and the skin epithelial layer thickness was measured using the Image J program. As a result, the average thickness of the epithelial layer was 23.8 µm in the non-psoriasis control group (rtTA), and the average thickness of the epithelial layer was 104.5 µm in the psoriasis-induced rtTA-Peli1 group (rtTA-Peli1 Vehicle), and thus, it was confirmed that the thickness of the epithelial layer in the psoriasis-induced rtTA-Peli1 group (rtTA-Peli1 Vehicle) was increased by about 4.4 times compared to the control group (FIGS. 19a and 19b). On the other hand, the average thicknesses of the epithelial layers were about 97.9 µm, 86.3 µm and 73.3 µm in the groups administered intravenously with 1, 3 and 10 mg/kg of the PD-L1-hyFc21 fusion protein, respectively, and thus, it was confirmed that there was an effect of reducing the thickness of the epithelial layer in a dose-dependent manner, compared to the psoriasis-induced rtTA-Peli1 group (rtTA-Peli1 Vehicle) (FIGS. 19a and 19b).

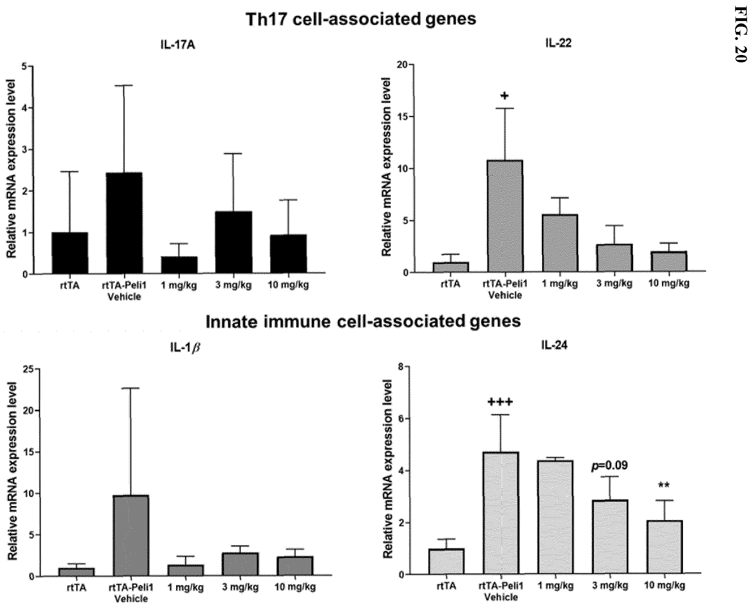

In addition, after intravenous administration of the PD-L1-hyFc21 fusion protein to the rtTA-Peli1 psoriasis mouse model, qRT-PCR was performed to confirm changes in the mRNA expressions of Th17 cell-associated genes such as IL-17A and IL-22 and innate immune cell-associated genes such as IL-1β and IL-24. As a result, the psoriasis-induced rtTA-Peli1 group (rtTA-Peli1 Vehicle) showed the increased mRNA expressions of Th17 cell-associated genes such as IL-17A and IL-22 compared to the control group (rtTA), whereas it was confirmed that the mRNA expressions of the Th17 cell-associated genes were reduced in the group intravenously administered with the PD-L1-hyFc21 fusion protein (FIG. 20). In addition, similarly, the mRNA expressions of the innate immune cell-associated genes such as IL-1β and IL-24 increased in the psoriasis-induced rtTA-Peli1 group (rtTA-Peli1 Vehicle) compared to the control group (rtTA), whereas the mRNA expressions of the Th17 cell-associated genes showed a tendency to decrease in the group intravenously administered with the PD-L1-hyFc21 fusion protein (FIG. 20). In particular, the expression of IL-24 mRNA was significantly reduced in the group intravenously administered with 10 mg/kg of the PD-L1-hyFc21 fusion protein (FIG. 20).

From the above results, it was confirmed that since the PD-L1-hyFc21 fusion protein inhibits the proliferation of CD4 T cells, which is an important factor in the pathogenesis of autoimmune diseases such as psoriasis, through subcutaneous administration or intravenous administration, and has an effect of suppressing the expression of inflammatory cytokines accompanying the lesion, it can be used as a therapeutic agent for immune diseases such as psoriasis.

## Claims

1. A fusion protein, comprising programmed cell death-ligand 1 (PD-L1) protein and a modified immunoglobulin Fc region.

2. The fusion protein of claim 1, wherein the PD-L1 protein is an extracellular domain of PD-L1 protein or a fragment thereof.

3. The fusion protein of claim 2, wherein the PD-L1 protein consists of an amino acid sequence selected from the group consisting of SEQ ID NO: 1 to SEQ ID NO: 6, or a polypeptide having at least 70% homology to an amino acid sequence selected from the group consisting of SEQ ID NO: 1 to SEQ ID NO: 6.

4. The fusion protein of claim 1, wherein the PD-L1 protein is fused to the N-terminus or C-terminus of the modified immunoglobulin Fc region.

5. The fusion protein of claim 1, wherein the PD-L1 protein is linked to the modified immunoglobulin Fc region by a linker peptide.

6. The fusion protein of claim 5, wherein the linker peptide consists of at least one amino acid sequence selected from the group consisting of GGGSGGS (SEQ ID NO: 10), AAGSGGGGGSGGGGSGGGGS (SEQ ID NO: 17), GGSGG (SEQ ID NO: 18), GGSGGSGGS (SEQ ID NO: 19), GGGSGG (SEQ ID NO: 20), (G4S)n (n is an integer from 1 to 10), (GGS)n (n is an integer from 1 to 10), (GS)n (n is an integer from 1 to 10), (GSSGGS)n (n is an integer from 1 to 10), KESGSVSSEQLAQFRSLD (SEQ ID NO: 21), EGKSSGSGSESKST (SEQ ID NO: 22), GSAGSAAGSGEF (SEQ ID NO: 23), (EAAAK)n (n is an integer from 1 to 10), CRRRRRREAEAC (SEQ ID NO: 24), A(EAAAK)4ALEA(EAAAK)4A, GGGGGGGG (SEQ ID NO: 25), GGGGGG (SEQ ID NO: 26), AEAAAKEAAAAKA (SEQ ID NO: 27), PAPAP (SEQ ID NO: 28), (Ala-Pro)n (n is an integer from 1 to 10), VSQTSKLTRAETVFPDV (SEQ ID NO: 29), PLGLWA (SEQ ID NO: 30), TRHRQPRGWE (SEQ ID NO: 31), AGNRVRRSVG (SEQ ID NO: 32), RRRRRRRR (SEQ ID NO: 33), GFLG (SEQ ID NO: 34) and GSSGGSGSSGGSGGGDEADGSRGSQKAGVDE (SEQ ID NO: 35).

7. The fusion protein of claim 6, wherein the linker peptide consists of an amino acid sequence of GGGSGGS (SEQ ID NO: 10).

8. The fusion protein of claim 1, wherein the modified immunoglobulin Fc region is any one of the Fc regions of IgG1, IgG2, IgG3, IgD and IgG4, or a combination thereof.

9. The fusion protein of claim 1, wherein the modified immunoglobulin Fc region comprises a hinge region, a CH2 domain and a CH3 domain from an N-terminal to C-terminal direction,
wherein the hinge region comprises a human IgG1 hinge region,
wherein the CH2 domain comprises portions of the amino acid residues of the CH2 domains of human IgD and human IgG4, and
wherein the CH3 domain comprises a portion of the amino acid residues of the CH3 domain of human IgG4.

10. The fusion protein of claim 1, wherein the modified immunoglobulin Fc region consists of the amino acid sequence of SEQ ID NO: 11.

11. The fusion protein of claim 10, wherein the modified immunoglobulin Fc region comprises an IgG1 hinge region which consists of the amino acid sequence of SEQ ID NO: 16.

12. The fusion protein of claim 1, wherein the fusion protein forms dimerization.

13. The fusion protein of claim 1, wherein the fusion protein consists of the amino acid sequence of SEQ ID NO: 12 or SEQ ID NO: 13.

14. A nucleic acid molecule encoding the fusion protein according to any one of claims 1 to 13.

15. An expression vector, comprising the nucleic acid molecule of claim 14.

16. A host cell, comprising the expression vector of claim 15.

17. A pharmaceutical composition for preventing or treating immune disease, comprising the fusion protein according to any one of claims 1 to 13 as an active ingredient.

18. The pharmaceutical composition of claim 17, further comprising a pharmaceutically acceptable carrier.

19. The pharmaceutical composition of claim 17, wherein the immune disease is selected from the group consisting of autoimmune disease and inflammatory disease.

20. The pharmaceutical composition of claim 19, wherein the autoimmune disease is selected from the group consisting of type 1 diabetes, alopecia areata, antiphospholipid antibody syndrome, rheumatoid arthritis, psoriasis, psoriatic arthritis, multiple sclerosis, systemic lupus erythematosus, inflammatory bowel disease, Addison's disease, Graves' disease, Sjogren's syndrome, Guillian-Barre syndrome, Hashimoto's thyroiditis, Myasthenia gravis, inflammatory myopathy, autoimmune vasculitis, autoimmune hepatitis, hemolytic anemia, idiopathic thrombocytopenic purpura, primary biliary cirrhosis, scleroderma, vitiligo, pernicious anemia and celiac disease.

21. The pharmaceutical composition of claim 19, wherein the inflammatory disease is selected from the group consisting of arthritis, ankylosing spondylitis, reactive arthritis, Reiter's syndrome, crystal arthropathies, Lyme disease, polymyalgia rheumatica, systemic sclerosis, polymyositis, dermatomyositis, polyarteritis nodosa, Wegener's granulomatosis, Churg-Strauss syndrome, sarcoidosis, atherosclerotic vascular disease, atherosclerosis, ischaemic heart disease, myocardial infarction, stroke, peripheral vascular disease, uveitis, corneal disease, iritis, iridocyclitis and cataracts.
